(19) Europäisches Patentamt
European Patent Office
Office européen des brevets

(11) EP 2 198 892 A1

(12) EUROPÄISCHE PATENTANMELDUNG

(43) Veröffentlichungstag:
23.06.2010 Patentblatt 2010/25

(51) Int Cl.:
A61L 15/18 (2006.01)    A61L 15/46 (2006.01)
A61L 15/60 (2006.01)

(21) Anmeldenummer: 08021914.0

(22) Anmeldetag: 17.12.2008

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Süd-Chemie AG**
**80333 München (DE)**

(72) Erfinder:
• **Sohling, Ulrich Dr.**
**85356 Freising (DE)**

• **Kuhn, Hubert Dr.**
**42697 Solingen (DE)**
• **Thie, Gordon**
**45527 Hattingen (DE)**

(74) Vertreter: **Stolmár, Matthias et al**
**Stolmár Scheele & Partner**
**Patentanwälte**
**Blumenstrasse 17**
**80331 München (DE)**

(54) **Verwendung eines Trägers zur Inhibierung der Aktivität von Urease, sowie Verfahren zu dessen Herstellung und absorbierender Artikel**

(57)    Die vorliegende Erfindung betrifft eine Verwendung eines festen, teilchenförmigen Trägers zur Inhibierung der Aktivität von Urease in einem Urease enthaltenden fluiden Medium, wobei das feste teilchenförmige Trägermaterial mit mindestens einer Zinkionen und/oder Kupfer(II)-Ionen enthaltenden Lösung vor dem Inkontaktbringen mit dem Urease enthaltenden fluiden Medium behandelt wurde, sowie ein Verfahren zur Herstellung einer Ureaseinhibitor-Träger-Zusammensetzung und absorbierende Artikel.

**Urease-Inhibierung, Vergleich ZnO-Adsorbentien**

Fig.1

EP 2 198 892 A1

**Beschreibung**

Die vorliegende Erfindung betrifft eine Verwendung eines festen teilchenförmigen Trägers zur Inhibierung der Aktivität von Urease in einem Urease enthaltenden fluiden Medium, sowie ein Verfahren zur Herstellung einer Urease-einhibitor-Träger-Zusammensetzung und absorbierende Artikel.

Eine Verhinderung oder Verringerung von Geruchsbelastungen, die von menschlichen oder tierischen Körperfluiden ausgehen, wird bei zahlreichen technischen Anwendungen angestrebt. Anwendungen von beträchtlicher wirtschaftlicher Bedeutung sind insbesondere die Herstellung von Hygieneartikeln, beispielsweise von Windeln, Binden und Einlagen. Zudem ist auch eine Verhinderung oder Verringerung von Geruchsbelastungen, die von Sanitäranlagen und Ställen ausgehen, von technischem Interesse und wirtschaftlicher Bedeutung.

Insbesondere können Geruchsbelastungen von Urin und dessen Zersetzungsprodukten ausgehen. Urin umfasst als Hauptbestandteil Harnstoff, wobei dieser innerhalb und insbesondere außerhalb des Körpers durch das Enzym Urease in Ammoniak umgewandelt wird. Ammoniak weist einen stechend riechenden Geruch auf, der von Menschen im Allgemeinen als unangenehm wahrgenommen wird. Eine Verringerung oder Verhinderung der von Urin und dessen Zersetzungsprodukten ausgehenden Geruchsbelastung ist insbesondere für erkrankte und/oder unter Inkontinenz leidende Personen von großer Bedeutung, da ein Austritt von Urin auf diese Weise nicht oder in geringerem Maße von der Umgebung wahrgenommen wird. Da Harnstoff in Urin jedoch in vergleichsweise großen Mengen vorliegt, ist eine Aufnahme des Ammoniaks oder des Harnstoffs durch adsorptive Vorgänge nicht oder nur schwer möglich, da hierzu sehr große Mengen an Adsorbens erforderlich sind.

Ein möglicher Ansatz zur Verringerung oder Verhinderung einer Geruchsbelästigung durch Urin oder durch die aus Harnstoff gebildeten Folgeprodukte ist ein Zusatz von üblicherweise als wohlriechend angesehenen Duftstoffen zu Hygieneartikeln. Insbesondere können zu diesem Zweck eingekapselte oder umhüllte Duftstoffe eingesetzt werden, die erst durch Einwirkung von Feuchtigkeit aktiviert und freigesetzt werden. Dieser Ansatz ermöglicht jedoch nicht, die Geruchsbelastung bereits vor oder bei ihrer Entstehung zu vermeiden oder zu verringern, sondern dient lediglich zur Verschleierung des sich bildenden ammoniakalischen Geruchs.

Ein weiterer Ansatz beruht darauf einen Urease-Inhibitor in wirksamen Mengen mit Harnstoff-haltigen Körperflüssigkeiten in Kontakt zu bringen. Beispielsweise beschreibt die US 2003/0220211 A1 ein Verfahren, bei dem es möglich ist, eine unerwünschte Ammoniakbildung durch eine Inhibierung der Urease zu verringern oder zu vermeiden. Hierzu werden Metallkomplexe eingesetzt, die so aufgebaut sind, dass eine freie Koordinationsstelle verbleibt, die an das aktive Zentrum der Urease binden kann. Durch Einstellung einer hohen Komplexbildungskonstante wird verhindert, dass die Urease noch Harnstoff in Ammoniak umsetzen kann. Bei diesem Verfahren wird jedoch mit Komplexbildnern gearbeitet, deren aktive Gruppierungen zum Teil mit EDTA verwandt sind. Erfahrungsgemäß sind solche Verbindungen nur schwer biologisch abbaubar und reichern außerdem giftige Schwermetallionen im Sediment der Gewässer an. Aus diesen Gründen sollte eine Verwendung derartiger Verbindungen im Bereiche der Haushalts- und Hygieneartikel vermieden werden. Ergänzend wird hierzu auf die Schrift WO 02/47472 verwiesen.

Weiterhin offenbart die DE 100 16 488 A1 ein Mittel zur Verbesserung der Stickstoffausnutzung mineralischer und/oder organischer harnstoffhaltiger Düngemittel. Bei harnstoffhaltigen Düngemitteln ist eine schnelle unkontrollierte Urease katalysierte Harnstoffhydrolyse unerwünscht, so dass niedermolekulare Verbindungen zugesetzt werden, um die Urease zu inhibieren. Hierzu können gemäß dieser Schrift insbesondere Phosphorsäureamide verwendet werden. Derartige Verbindungen sind jedoch für Haushalts- und Hygieneanwendungen wegen ihrer reizenden Eigenschaften nicht geeignet.

Zudem beschreibt die WO 2007/085531 A2 geruchsverhindernde, wasserabsorbierende Zusammensetzungen, die auf Superabsorbern basieren. Diesen Zusammensetzungen wird Urease-Inhibitor, beispielsweise ein Thiophosphorsäureamid, zugesetzt.

Die im Stand der Technik beschriebenen Verfahren, beruhen jedoch oftmals auf einer Verwendung von Verbindungen oder Zusammensetzungen, die aus Kosten-, Gesundheits- oder Umweltgründen für Haushalts- und Hygieneanwendungen nicht oder weniger geeignet sind oder lehren unter wirtschaftlichen und/oder verfahrenstechnischen Aspekten ungünstige Vorgehensweisen.

Eine Aufgabe der vorliegenden Erfindung ist daher die Bereitstellung eines Mittels oder eines alternativen Mittels, das eine von Urin und dessen Folgeprodukten ausgehende Geruchsbelastung verhindert oder zu deren Verringerung beiträgt. Darüber hinaus sollte ein derartiges Mittel auch zur Behandlung fester Oberflächen, wie beispielsweise von Vliesmaterial in Windeln, eingesetzt werden können. Um eine kostengünstige Bereitstellung eines derartigen Mittels, sowie der damit hergestellten, in großen Mengen anfallenden Hygieneprodukte, wie beispielsweise Windeln und Binden, zu ermöglichen, sollte die Herstellung der erfindungsgemäßen Produkte zudem nur zu geringen zusätzlichen Arbeits- und Materialkosten führen.

Gelöst wird diese Aufgabe durch eine Verwendung eines festen, teilchenförmigen Trägers zur Inhibierung der Aktivität von Urease in einem Urease enthaltenden fluiden Medium, wobei der feste teilchenförmige Träger mit mindestens einer Zinkionen und/oder Kupfer(II)-Ionen enthaltenden Lösung vor dem Inkontaktbringen mit dem Urease enthaltenden fluiden Medium behandelt wurde, und wobei die mindestens eine Zinkionen und/oder Kupfer(II)-Ionen enthaltende Lösung eine Lösung mindestens einen Zinksalzes und/oder mindestens einen Kupfer(II)-Salzes umfasst, wobei das mindestens eine Zinksalz und das mindestens eine Kupfer(II)-Salz vorzugsweise frei von dreizähnigen oder mehr als dreizähnigen Chelat-Liganden sind.

Weiterhin lehrt die vorliegende Erfindung ein Verfahren zur Herstellung einer Ureaseinhibitor-Träger-Zusammensetzung, umfassend die Schritte: a. Bereitstellen mindestens eines festen teilchenförmigen Trägers; b. Bereitstellen mindestens einer Zinkionen und/oder Kupfer(II)-Ionen enthaltenden Lösung; c. Behandeln des mindestens einen festen teilchenförmigen Trägers mit der mindestens einen Zinkionen und/oder Kupfer(II)-Ionen enthaltenden Lösung, vorzugsweise in Abwesenheit eines Urease enthaltenden fluiden Mediums; und d. optional Trocknen des gemäß Schritt c. behandelten festen teilchenförmigen Trägers.

Zudem stellt die vorliegende Erfindung einen absorbierenden Artikel bereit, insbesondere Windel, Hygienebinde, Windeleinlage, Binde, Einlage, Tuch, Betteinlage, Inkontinenzartikel, Haustierstreu, Streu zum Ausbringen in Nutztierställen, umfassend, mindestens ein Absorbens, vorzugsweise mindestens einen Superabsorber, wobei der Superabsorber vorzugsweise ausgewählt ist aus der Gruppe, bestehend aus Polyacrylaten, Polyacrylsäuren, Carboxy-Cellulosen, insbesondere Carboxymethylcellulosen, und deren Gemischen, sowie einen mit mindestens einer Zinkionen und/oder Kupfer(II)-Ionen enthaltenden Lösung behandelten festen teilchenförmigen Träger.

Bevorzugte Ausführungsformen sind jeweils in den Unteransprüchen angegeben.

Die beigefügten Figuren dienen der Erläuterung der vorliegenden Erfindung ohne diese einzuschränken:

**Fig.en 1** bis **5** zeigen Diagramme, welche die erfindungsgemäße Verwendung eines mit Zinkionen behandelten Trägers erläutern, bei der eine Verhinderung oder Verringerung der Bildung von Ammoniak aus Harnstoff in Gegenwart von Urease erfolgt.

Während der zahlreichen Versuche, die zu der vorliegenden Erfindung führten, wurde überraschenderweise festgestellt, dass ein fester, teilchenförmiger Träger, der mit einer Zinksalzlösung und/oder Kupfer(II)-Salzlösung behandelt wurde, hervorragende Eigenschaften zum Inhibieren einer Ureaseaktivität aufweist und hierdurch eine Bildung von unangenehmen und auffälligen Geruchsbelastungen wirksam verhindert. In besonderem Maße von Vorteil ist, dass die vorliegende Erfindung eine wirksame Inhibierung der Urease-Aktivität bereits bei sehr geringen Mengen an Zink und Trägerstoffen bereitstellt, wodurch sowohl die Ressourcen geschont werden können, als auch dem Verbraucher kostengünstige und wirksame Produkte zur Verfügung gestellt werden können. Darüber hinaus ermöglicht die vorliegende Erfindung auch eine Behandlung von festen Oberflächen, beispielsweise von Vliesmaterial in Windeln.

Ohne dass die vorliegende Erfindung auf die Richtigkeit der nachstehenden Theorie beschränkt wäre, wird angenommen, dass die bei der erfindungsgemäßen Verwendung erfolgende weitgehende Inhibierung der Urease durch eine Aufnahme von Zink- und/oder Kupfer(II)-Ionen in die aktive Tasche der Urease und die hieraus resultierende Blockierung der aktiven Tasche hervorgerufen wird. Insbesondere ist eine mögliche Erklärung, dass die Blockierung der aktiven Tasche durch einen Austausch des in der aktiven Tasche vorhandenen $Ni^{2+}$ durch $Zn^{2+}$ und/oder $Cu^{2+}$ hervorgerufen wird, wobei Urease im aktiven Zentrum $Ni^{2+}$-Ionen aufweist.

Weiterhin wird angenommen, ohne dass die vorliegende Erfindung auf die Richtigkeit der nachstehenden Theorie beschränkt wäre, dass zu der Inhibierung der Urease insbesondere beiträgt, dass Urease an einen mit $Zn^{2+}$ und/oder $Cu^{2+}$ - behandelten, insbesondere adsorbierte $Zn^{2+}$ - und/oder $Cu^{2+}$ - Ionen aufweisenden, Träger adsorbiert wird, wobei die Urease durch die auf dem Träger adsorbierten $Zn^{2+}$ - und/oder $Cu^{2+}$ - Ionen inhibiert wird. In Gegenwart von Komplexbildnern, wie beispielsweise Polycarboxylaten, kann auf diese Weise eine Inhibierung der Urease gelingen, ohne dass die freien $Zn^{2+}$ - und/oder $Cu^{2+}$ - Ionen in der Lösung nach einer sehr kurzen Zeitspanne durch die Komplexbildner gebunden werden und die Urease nicht mehr inhibieren können.

Durch eine Adsorption der Urease auf einem Träger, beispielsweise in den Poren und/oder Zwischenschichten des Trägers, wird die Migration der gebundenen Zink- und/oder Kupferionen in das Medium, insbesondere hin zu Zinkionen und/oder Kupfer(II)-Ionen abfangenden Komponenten, wie beispielsweise Komplexbildnern, stark verlangsamt. Auf diese Weise verbleibt die Urease deutlich länger blockiert, als bei einer Zugabe von Zinksalzen und/oder Kupfer(II)-Salzen oder deren Lösungen als solchen. Zusätzlich kann angenommen werden, dass die hohe lokale Zink- und/oder Kupfer(II)-Ionenkonzentration an der Oberfläche der Träger und damit in räumlicher Nähe zu einer am Träger adsorbierten oder in der Umgebung des Trägers vorliegenden Urease die Inhibierung der Urease weiter fördert.

Die vorliegende Erfindung lehrt eine Verwendung eines festen teilchenförmigen Trägers zur Inhibierung der Aktivität von Urease in einem Urease enthaltenden fluiden Medium, wobei der feste teilchenförmige Träger mit mindestens einer Zinkionen und/oder Kupfer(II)-Ionen enthaltenden Lösung vor dem Inkontaktbringen mit dem Urease enthaltenden fluiden Medium behandelt wurde.

Der Begriff "Inhibierung der Aktivität von Urease" umfasst sowohl eine Verringerung, als auch eine vollständige Verhinderung der Aktivität einer oder mehrerer Ureasen, beispielsweise von Urease bakterieller Herkunft oder von Urease aus anderen biologischen Quellen. Die Urease kann von beliebiger Herkunft, insbesondere beliebiger bakterieller, tierischer oder pflanzlicher Herkunft sein.

Die Inhibierung erfolgt in einem Urease enthaltenden fluiden Medium, das heißt in einem Medium, das fließfähig, insbesondere flüssig sein kann, und das beispielsweise als eine Lösung, eine Suspension, eine Emulsion oder ein Aerosol vorliegen kann. Insbesondere kann das fluide Medium auch ungelöste Partikel oder Objekte enthalten. Vorzugsweise handelt es sich bei dem fluiden Medium um menschlichen oder tierischen Urin oder um ein Gemisch, das menschlichen oder tierischen Urin umfasst, beispielsweise um ein Urin umfassendes Gemisch mit Wasser, und/oder mit weiteren menschlichen oder tierischen Ausscheidungen oder Körperflüssigkeiten, wie beispielsweise Stuhlgang, Blut, usw., und/oder Streu- und/oder Bodenbedeckungsmitteln für Ställe und/oder Tierkäfige und/oder saugfähigen Materialien, wie beispielsweise Papier, etc. Der Begriff "Urin", wie er im Rahmen der vorliegenden Anmeldung verwendet wird, umfasst jegliche menschliche oder tierische Ausscheidung, die Wasser und Harnstoff umfasst. Bei tierischem Urin kann es sich insbesondere um Säugetier-Urin handeln.

Die mindestens eine Zinkionen und/oder Kupfer(II)-Ionen enthaltende Lösung umfasst eine Lösung mindestens eines Zinksalzes und/oder mindestens eines Kupfer(II)-Salzes, wobei als Zinksalz und/oder Kupfer(II)-Salz von einem Fachmann auf Basis des allgemeinen Fachwissens und der Lehre der vorliegenden Erfindung jegliches geeignete Zinksalz und/oder Kupfer(II)-Salz ausgewählt werden kann.

Gemäß einer bevorzugten erfindungsgemäßen Ausführungsform kann das mindestens eine Zinksalz und das mindestens eine Kupfer(II)-Salz jeweils eine Löslichkeit in Wasser von mehr als $10^{-10}$ mol/l, vorzugsweise von mehr als $10^{-8}$ mol/l, bevorzugt von mehr als $10^{-5}$ mol/l, weiter bevorzugt von mehr als $10^{-2}$ mol/l, noch weiter bevorzugt von mehr als $10^{-1}$ mol/l, noch weiter bevorzugt von mehr als 0,5 mol/l, insbesondere von mehr als 1 mol/l aufweisen.

Die Ermittlung der Löslichkeit eines Zink- oder Kupfer(II)-Salzes kann der Literatur entnommen werden, oder, nach einer Ausführungsform, wie im Methodenteil erläutert erfolgen. Ohne dass die vorliegende Erfindung auf die Richtigkeit der nachstehenden Annahme beschränkt wäre, wird angenommen, dass eine Löslichkeit des mindestens einen Zinksalzes beziehungsweise des mindestens einen Kupfer(II)salzes in Wasser von mehr als $10^{-10}$ mol/l oder über einen der vorstehenden bevorzugten Mindestwerte hinaus dazu beiträgt, dass die Inhibierung der Urease in vorteilhafter Weise durch zumindest teilweise gelöst vorliegende Zinkionen beziehungsweise Kupfer(II)-Ionen erfolgen kann.

Bei dem mindestens einen Zinksalz und/oder Kupfer(II)-Salz kann es sich gemäß einer bevorzugten erfindungsgemäßen Ausführungsform zudem um Zinksalz(e) beziehungsweise Kupfer(II)-Salze handeln, die jeweils vorzugsweise keine dreizähnigen und keine mehr als dreizähnigen Chelat-Liganden aufweisen, sowie weiter bevorzugt frei von Chelat-Liganden sind, das heißt keine Chelat-Liganden umfassen. Für diese erfindungsgemäßen Ausführungsformen wurden überraschend verbesserte Wirksamkeiten festgestellt. Der Begriff "Chelat-Ligand" ist einem Fachmann bekannt und beschreibt Liganden, die sich mit zwei oder mehr komplexbildenden Atomen an ein Zentralatom unter Schließung eines oder mehrerer Ringe anzulagern vermögen. Ein Ligand der in einem gebildeten Komplex nur eine Koordinationsstelle besetzen kann wird als einzähniger Ligand bezeichnet, während ein Ligand der in einem gebildeten Komplex mehrere (z.B. drei) Koordinationsstellen besetzen kann, als mehrzähniger (z.B. dreizähniger) Ligand bezeichnet wird. Der Begriff mehr als dreizähniger Ligand bezeichnet vierzähnige, fünfzähnige, sechszähnige, und höherzähnige Liganden.

Insbesondere kann bei einigen Anwendungen das mindestens eine Zinksalz und/oder mindestens eine Kupfer(II)-Salz ein- und/oder zweizähnige Liganden umfassen. Bei einigen erfindungsgemäßen Ausführungsformen kann anwendungsspezifisch bevorzugt sein, dass das mindestens eine Zinksalz und/oder mindestens eine Kupfer(II)-Salz Anionen umfasst oder ausschließlich aufweist, die in Komplexen lediglich eine Koordinationsstelle besetzen können und als einzähnige Liganden bezeichnet werden, wie beispielsweise Halogenidanionen, etc.. Beispiele und Erläuterungen zu den Begriffen Chelatligand, sowie ein-, zwei- oder mehrzähnige Liganden finden sich beispielsweise in den Lehrbüchern "Holleman-Wiberg, Lehrbuch der Anorganischen Chemie" von N. Wiberg, 91.-100. Aufl., Walter de Gruyter & Co., 1985, ISBN 3-11-007511-3, S. 969 bis 983, sowie "Anorganische Chemie" von E. Riedel, Walter de Gruyter& Co., 1988, ISBN 3-11-010162-9, S. 597 -606.

Durch die Bereitstellung eines mit Zinkionen und/oder Kupfer(II)-Ionen behandelten Trägers gelingt eine wirksame Inhibierung eines Enzyms, der Urease, ohne dass oftmals sehr kostenaufwändig herzustellende Chelatliganden und insbesondere drei- und höherzähnige Chelatliganden eingesetzt werden müssen. Zudem handelt es sich bei Chelatliganden häufig um reizende Verbindungen und/oder um Verbindungen, die als solche oder in Form von Komplexen biologisch nur schwer abbaubar und wenig umweltverträglich sind. Da die erfindungsgemäßen absorbierenden Artikel, beispielsweise Windeln und Binden, einen signifikanten Anteil am Hausmüll darstellen, können die erfindungsgemäßen absorbierenden Artikel einen deutlichen Vorteil aufweisen.

Zudem können die eine oder die mehreren Zinkionen und/oder Kupfer(II)-Ionen enthaltenden Lösungen, die zur Behandlung des Trägers verwendet werden, frei von drei- oder höherzähnigen Chelatliganden (in freier Form und/oder in Form von Komplexen), insbesondere frei von Chelatliganden (in freier Form und/oder in Form von Komplexen) sein.

Gemäß einer Ausführungsform kann der feste teilchenförmige Träger kein Carboxylgruppen aufweisendes Polymer darstellen oder dieses umfassen, bevorzugt kein Carboxyl- oder Sulfonylgruppen aufweisendes Polymer darstellen oder dieses umfassen.

Der Begriff "Polymer" umfasst im Rahmen der vorliegenden Anmeldung jegliches Molekül, das mehr als 20, vorzugsweise mehr als 50 polymerisierte Monomereinheiten umfasst, wobei die Monomereinheiten die gleiche oder eine unterschiedliche chemische Struktur aufweisen können.

Der Begriff "Carboxylgruppen aufweisendes Polymer" beschreibt jegliches Polymer das mindestens eine Carboxylgruppe, vorzugsweise mindestens zwei Carboxylgruppen, bevorzugt mindestens 20 Carboxylgruppen, insbesondere mindestens 50 Carboxylgruppen aufweist. Diese Polymere können auch vollständig oder teilweise in ionischer Form beziehungsweise als Salze vorliegen.

Der Begriff "Carboxyl- oder Sulfonylgruppen aufweisendes Polymer" beschreibt jegliches Polymer das mindestens eine Carboxylgruppe und/oder mindestens eine Sulfonylgruppe, vorzugsweise mindestens zwei Carboxylgruppen und/oder mindestens zwei Sulfonylgruppen, bevorzugt mindestens 20 Carboxylgruppen und/oder mindestens 20 Sulfonylgruppen, insbesondere mindestens 50 Carboxylgruppen und/oder mindestens 50 Sulfonylgruppen aufweist. Diese Polymere können auch vollständig oder teilweise in ionischer Form beziehungsweise als Salze vorliegen.

Der Begriff "Hydroxy-, Carboxyl- oder Sulfonylgruppen umfassendes Polymer" beschreibt jegliches Polymer das mindestens eine Hydroxy- oder Carboxyl- oder Sulfonylgruppe, vorzugsweise mindestens zwei Hydroxy- oder mindestens zwei Carboxyl-, oder mindestens zwei Sulfonylgruppen, umfasst, bevorzugt mindestens zwanzig Hydroxy- oder mindestens zwanzig Carboxyl-, oder mindestens zwanzig Sulfonylgruppen, insbesondere mindestens 50 Hydroxy- oder mindestens 50 Carboxyl-, oder mindestens 50 Sulfonylgruppen, umfasst. Insbesondere kann es sich bei dem Hydroxy-, Carboxyl- oder Sulfonylgruppen umfassenden Polymer um ein aus Cellulose oder chemisch modifizierter Cellulose bestehendes oder diese umfassendes Polymer oder um ein aus Polyacrylsäure oder chemisch modifizierter Polyacrylsäure bestehendes oder diese umfassendes Polymer handeln.

Sehr gute Ergebnisse bei der Inhibierung der Ureaseaktivität können erhalten werden, wenn beispielsweise Zinkacetat, Zinkchlorid, Zinkchlorat, Zinkbromid, Zinksulfat, Zinkborat, Zinkbromat, Zinkbromid, Zinksulfat, Zinkcarbonat, Zinkhydroxid, Zinkabietat, Zinksalze von Fettsäuren, Zinkacetylacetonat, Zinksulfide, Zinknitrat, Zinkphosphat, Zinkcitrat, Zinkchromat, Zinkiodid, Zinkfluorid, Zinkgluconat, Zinkhydroxy-carbonat, Zinkborat, Zinkcarbamat, Zinkphthalat, Zinkmaleat, Zinksalicylat, Zinkgluconat, Zinktartrat, Zinkisophthalat, Zinkorthophthalat, Zinkadipate, Zinkglutamate, Zinkphosphit, Zinksilikat, Zinktrimellithat, Zinkpyromellithat, Zinkterephthalat, Zinkfumarat , Zinkorthophosphat, Zinkfluoroborat, Zinklactat, Zinkchromat, Kupfer(II)acetat, Kupfer(II)chlorid, Kupfer(II)chlorat, Kupfer(II)bromid, Kupfer(II)sulfat, Kupfer(II)borat, Kupfer(II)bromat, Kupfer(II)bromid, Kupfer(II)-sulfat, Kupfer(II)carbonat, Kupfer(II)hydroxid, Kupfer (II)-abietat, Kupfer(II)salze von Fettsäuren, Kupfer(II)acetylacetonat, Kupfer(II)sulfide, Kupfer(II)nitrat, Kupfer(II)-phosphat, Kupfer(II)citrat, Kupfer(II)chromat, Kupfer(II)-iodid, Kupfer(II)fluorid, Kupfer(II)gluconat, Kupfer(II)hydroxycarbonat, Kupfer(II)borat, Kupfer(II)carbamat, Kupfer-(II)phthalat, Kupfer(II)maleat, Kupfer(II)salicylat, Kupfer-(II)gluconat, Kupfer (II)tartrat, Kupfer(II)isophthalat, Kupfer(II)orthophthalat, Kupfer(II)adipate, Kupfer(II)glutamate, Kupfer(II)phosphit, Kupfer(II)silikat, Kupfer(II) trimellithat, Kupfer(II)pyromellithat, Kupfer(II)terephthalat, Kupfer(II)fumarat, Kupfer(II)orthophosphat, Kupfer(II)fluoro-borat, Kupfer(II)lactat, Kupfer(II)chromat, Zinkricinoleat, Kupfer(II)ricinoleat und Gemische hiervon zur Behandlung des Trägers eingesetzt werden.

Gemäß einer möglichen Ausführungsform kann der Träger, der beispielsweise Zinkoxid (bzw. Kupfer(II)oxid) umfasst oder hieraus besteht, mit einer Lösung, die das gleiche Kation umfasst, das heißt mit einer Zinkkationen (bzw. Kupfer(II)-Kationen) umfassenden Lösung, beispielsweise auch einer Kupfer(II)oxid (bzw. Zinkoxid-)Lösung, behandelt werden.

Die Zinksalze und/oder Kupfer(II)-Salze können jeweils auch in Form von Hydraten vorliegen. Die eine oder die mehreren Zinkionen und/oder Kupfer(II)-Ionen enthaltenden Lösungen, die zur Behandlung des Trägers verwendet werden, können Zinkoxid und/oder Zinksulfat umfassen, können jedoch auch frei von Zinkoxid und/oder Zinksulfat sein. Bei dem Zinksalz und/oder Kupfer(II)-Salz kann es sich um ein Zinksalz und/oder Kupfer(II)-Salz einer ungesättigten und/oder hydroxylierten Fettsäure, insbesondere Ricinolsäure handeln, anwendungsspezifisch kann jedoch, wenn erwünscht, auf eine Verwendung derartiger Zinksalze und/oder Kupfer(II)-Salze, insbesondere von Zinkricinoleaten, verzichtet werden. Als Fettsäuren werden im Rahmen der vorliegenden Anmeldung alle Carbonsäuren R-COOH bezeichnet, bei denen R ein gesättigter, geradkettiger oder verzweigter Rest mit 8 bis 30 Kohlenstoffatomen ist, sowie bei denen R ein ungesättigter, geradkettiger oder verzweigter Rest mit 8 bis 30 Kohlenstoffatomen und einer oder mehreren, vorzugsweise 1 bis 4 Doppelbindungen ist. Optional kann die Fettsäure mindestens eine OH-Gruppe, insbesondere 1 bis 6 OH-Gruppen, als Substituenten aufweisen. Wenn erwünscht, kann ein Zinksalz und/oder Kupfer(II)-Salz, das zur Behandlung des Trägers eingesetzt wird, kein organisches Anion, das heißt kein Anion, das sowohl Kohlenstoff, als auch Wasserstoff umfasst, enthalten. Es kann sich nach dieser Ausführungsform somit um anorganische Salze handeln.

Die Behandlung des festen, teilchenförmigen Trägers mit mindestens einer Zinkionen und/oder Kupfer(II)-Ionen enthaltenden Lösung kann nach einer möglichen Ausführungsform in Abwesenheit eines Hydroxy-, Carboxyl- oder Sulfonylgruppen umfassenden Polymers erfolgen oder nach einer weiteren möglichen Ausführungsform in Gegenwart eines Hydroxy-, Carboxyl- oder Sulfonylgruppen umfassenden Polymers erfolgen, wobei das Gewichtsverhältnis zwischen dem Gewicht an Hydroxy-, Carboxyl- oder Sulfonylgruppen umfassenden Polymer und dem Gewicht an Träger (vor einer Behandlung mit Zinkionen und/oder Kupfer(II)-Ionen enthaltender Lösung, wobei der Träger optional kein Carboxylgruppen umfassendes Polymer, weiter optional kein Hydroxy-, Carboxyl- oder Sulfonylgruppen umfassendes Polymer umfasst oder aus diesem besteht) nicht mehr als 2,8:1, vorzugsweise nicht mehr als 1:1, bevorzugt nicht mehr als 0,5:1, insbesondere nicht mehr als 0,3:1 betragen kann.

Die Behandlung des festen, teilchenförmigen Trägers mit mindestens einer Zinkionen und/oder Kupfer(II)-Ionen enthaltenden Lösung kann optional in Gegenwart eines oder mehrerer Fettsäureester oder Glycerin erfolgen, vorzugsweise beträgt das Gewichtsverhältnis zwischen dem Gesamtgewicht an Fettsäureestern und dem Gewicht an Träger (n) (vor einer Behandlung mit Zinkionen und/oder Kupfer(II)-Ionen enthaltender Lösung) nicht mehr als 1:4, vorzugsweise nicht mehr als 1:8, bevorzugt nicht mehr als 1:50. Vorzugsweise beträgt das Gewichtsverhältnis zwischen dem Gewicht an Glycerin und dem Gewicht an Träger(n) (vor einer Behandlung mit Zinkionen und/oder Kupfer(II)-Ionen enthaltender Lösung) nicht mehr als 1:10, bevorzugt nicht mehr als 1:50. Eine Behandlung des Trägers in Abwesenheit von einem oder mehreren Fettsäureestern und/oder Glycerin kann aus verfahrenstechnischen Gründen von Vorteil sein.

Ohne dass die vorliegende Erfindung auf die Richtigkeit der nachstehenden Annahme beschränkt wäre, wird angenommen, dass nach dieser möglichen Ausführungsform durch eine Behandlung des Trägers frei von oder in Gegenwart lediglich einer begrenzten Menge an Hydroxy-, Carboxyl- oder Sulfonylgruppen umfassendem Polymer und/oder frei von oder in Gegenwart lediglich einer begrenzten Menge an einem oder mehreren Fettsäureestern und/oder an Glycerin eine unbeeinträchtigte oder in ausreichendem Maße unbeeinträchtigte Anlagerung der Zinkionen und/oder Kupfer(II)-Ionen an oder Einlagerung der Zinkionen und/oder Kupfer(II)-Ionen an und/oder in den Trägerkörper erfolgen kann.

Auf diese Weise kann ein vorteilhafter erfindungsgemäßer Trägerkörper erhalten werden, der ein sehr gutes Urease-Inhibierungsvermögen aufweist. In hohem Maße von Vorteil ist hierbei, dass der Träger es ermöglicht, dass nicht nur die Zinkionen und/oder Kupfer(II)-Ionen auf oder in dem Trägerkörper vorliegen, sondern dass gleichzeitig auch der Harnstoff und/oder die Urease auf oder in dem Träger vorliegen können. Ohne dass die vorliegende Erfindung auf die Richtigkeit der nachstehenden Annahme beschränkt wäre, wird angenommen, dass die durch die Verwendung des Trägers vermittelte räumliche Nähe von Zinkionen und/oder Kupfer(II)-Ionen, Urease und/oder Harnstoff, die Inhibierung der Urease fördert. Insbesondere kann hierbei von Vorteil sein, dass die Zinkionen und/oder Kupfer(II)-Ionen in dem Trägerkörper lokal in vergleichsweise hohen Mengen vorliegen, wodurch der Reaktionsverlauf vorteilhaft beeinflusst werden kann.

Insbesondere kann die mindestens eine Zinkionen und/oder Kupfer(II)-Ionen enthaltende Lösung eine Lösung sein, die Wasser und/oder Alkohol umfasst, vorzugsweise eine wässrige Lösung sein. Gemäß einer Ausführungsform können Zink- und/oder Kupfer(II)-Verbindungen, insbesondere Fettsäuresalze, weiter insbesondere Zinkricinoleat, in Alkohol gelöst und dann aufgetragen werden. Sehr gute Ergebnisse können insbesondere erhalten werden, wenn als Alkohol Ethanol, Methanol, Propanol, Butanol oder Gemische hiervon oder Gemische dieser Alkohole mit Wasser verwendet werden.

Der Begriff eine "Zinkionen und/oder Kupfer(II)-Ionen enthaltende Lösung", wie er im Rahmen der vorliegenden Anmeldung verwendet wird, umfasst Lösungen, sowie zudem Zinkionen und/oder Kupfer(II)-Ionen enthaltende Suspensionen, insbesondere Suspensionen, in denen alle Zinkionen und/oder Kupfer(II)-Ionen enthaltenden Verbindungen, insbesondere ionischen Verbindungen, vollständig gelöst vorliegen, und Zinkionen und/oder Kupfer(II)-Ionen enthaltende Aerosole.

Eine Zinkionen und/oder Kupfer(II)-Ionen enthaltende Lösung kann darüber hinaus weitere Komponenten aufweisen, die auf Basis des allgemeinen Fachwissens und der vorliegenden Anmeldung ausgewählt werden können, wie beispielsweise anorganische und organische Säuren, anorganische und organische Basen, Salze, Puffergemische, anti-bakterielle Substanzen, Fungizide, Farbstoffe, Milchsäurebakterien, Duftstoffe, Tenside, usw..

Von Vorteil kann sein, wenn die Behandlung des Trägers mit einer Zinkionen und/oder Kupfer(II)-Ionen enthaltenden Lösung erfolgt, die eine Gesamtkonzentration an Zinkionen und Kupfer(II)-Ionen von beispielsweise mindestens 0,05 mol/l, vorzugsweise mindestens 0,1 mol/l, bevorzugt mindestens 0,15 mol/l, weiter bevorzugt mindestens 1 mol/l, noch weiter bevorzugt von mindestens 10 mol/l, insbesondere von 0,1 bis 10 mol/l aufweist. Optional kann die Behandlung des Trägers mit einer Zinkionen und/oder Kupfer(II)-Ionen enthaltenden Lösung ein- oder mehrmals wiederholt werden. Dies ermöglicht ein Aufbringen einer gewünschten Menge an Zinksalzen und/oder Kupfer(II)-Salzen auf den Träger auch bei einer Verwendung von Zinkionen und/oder Kupfer(II)-Ionen enthaltenden Lösungen, die lediglich eine geringe Konzentration an Zinkionen und/oder eine geringe Konzentration an Kupfer(II)-Ionen aufweisen. Wenn gewünscht kann die Behandlung des Trägers gleichzeitig und/oder zeitlich getrennt mit mehreren Zinkionen und/oder Kupfer(II)-Ionen enthaltenden Lösungen unterschiedlicher Zusammensetzung und/oder Konzentration erfolgen.

Als Träger kann jeglicher einem Fachmann bekannte Träger verwendet werden, wobei dieser von einem Fachmann auf Basis seines allgemeinen Fachwissens und der Lehre der vorliegenden Anmeldung ausgewählt werden kann. Gemäß einer Ausführungsform kann der Träger kein Carboxylgruppen aufweisendes Polymer umfassen oder aus diesem bestehen, weiter optional kann der Träger kein Carboxyl- oder Sulfonylgruppen aufweisendes Polymer umfassen oder aus diesem bestehen. Anwendungsspezifisch kann von Vorteil sein, wenn der Träger (vor einer Behandlung mit Zinkionen und/oder Kupfer(II)-Ionen enthaltender Lösung) nicht mehr als 95 Gew.-%, bevorzugt nicht mehr als 80 Gew.-%, insbesondere nicht mehr als 10 Gew.-% an organischen Komponenten, umfasst bezogen auf das Gesamtgewicht des Trägers vor der Behandlung mit Zinkionen und/oder Kupfer(II)-Ionen, wobei als organische Komponenten alle Komponenten angesehen werden, die sowohl Kohlenstoff, als auch Wasserstoff umfassen. Insbesondere kann der Träger (vor einer Behandlung mit Zinkionen und/oder Kupfer(II)-Ionen enthaltender Lösung) lediglich aus einem oder mehreren anorganischen Materialien bestehen.

Eine sehr gute Ureaseinhibierung kann nach einer erfindungsgemäßen Ausführungsform bei einer Kombination von Zinkoxid als Träger und einer Behandlung mit Zinkionen enthaltender Lösung erhalten werden.

Gemäß einer bevorzugten erfindungsgemäßen Ausführungsform kann der feste teilchenförmige Träger ein kumulatives Porenvolumen von beispielsweise mindestens 0,7, vorzugsweise von mindestens 0,8, weiter vorzugsweise von 0,75 bis 1,1, vorzugsweise von 0,8 bis 1,0 $cm^3/g$, für Porendurchmesser im Bereich von 1,7 bis 300 nm aufweisen.

Zudem kann nach einer bevorzugten erfindungsgemäßen Ausführungsform der feste teilchenförmige Träger eine BET-Oberfläche von mindestens 0,1 $m^2/g$, vorzugsweise von mindestens 5 $m^2/g$, bevorzugt von 0,1 bis 21 $m^2/g$, weiter bevorzugt von 19 bis 21 $m^2/g$, insbesondere etwa 20 $m^2/g$ aufweisen.

Bei der Auswahl der Träger können im Rahmen der vorliegenden Erfindung unterschiedliche Trägermaterialien zum Einsatz kommen. Anforderungen für den Träger sind, dass vorzugsweise Urease und/oder vorzugsweise Zinkionen und/oder Kupfer(II)-Ionen adsorbiert werden können. Da es sich bei der Urease um ein Enzym bzw. Protein handelt, gibt es unterschiedliche Mechanismen, die zur Adsorption dieses Enzyms auf einer Trägeroberfläche zum Tragen kommen können. In erster Näherung geht man zwar davon aus, dass ein Protein an Festkörperoberflächen dann besonders gut adsorbiert werden kann, wenn es bei einem pH-Wert vorliegt, bei dem die Ladung des Enzyms entgegengesetzt der Oberflächenladung ist. Diese Näherungsannahme lässt sich aber oftmals nicht verallgemeinern, weil es sich bei den Proteinen nicht um homogene Partikel mit homogener Ladungsverteilung handelt, sondern Bereiche mit unterschiedlichen Ladungseigenschaften oder Hydrophobizitäten vorliegen können. Zahlreiche Materialien ermöglichen eine Adsorption von Urease. Als Mechanismus werden hierbei nicht nur eine Ladungswechselwirkung, sondern auch hydrophobe Wechselwirkungen oder auch unspezifische Wechselwirkungen diskutiert. So wurde in dem Artikel "Sepiolite-supported Urease", Juan M. Garcia-Segura, Concepción Cid. Javier Martin de Llano and José G. Gavilanes, British Polymer Journal 19 (1987) 517-522, ausgeführt, dass sich das Tonmineral Sepiolith dazu eignet, Urease über hydrophobe Wechselwirkungen zu adsorbieren. Die adsorbierte Urease ist noch aktiv. Ein anderes Beispiel für einen Ureaseträger ist die Aktivkohle, wie sie in dem Artikel von Demirel et. al. eingesetzt wird (Günay Demirel, Güneri Akovali, Abdurrahman Tanyolac, Nesrin Hasirci, A Comparative Study of the Performance of Solid Supported and Soluble Urease for the Enzymatic Hydrolysis of Urea, J. Chem. Tech. Biotechnol. 1992, 55, 319-323). Auch hier geht man von einer hydrophoben Wechselwirkung zur Adsorption der Urease aus. Auch in diesem Fall ist die Urease noch aktiv. Ein Beispiel für die Adsorption und Immobilisierung von Urease an ein Tonmineral liefert die Publikation von A. Anita et. al., in der gezeigt wird, dass sich Urease an mit Aluminiumionen behandelte Vermiculite adsorbieren lassen (A. Anita, C.A. Sastry, M.A. Hashim, Immobilization of urease on vermiculite, Bioprocess Engineering 16 (1997) 375-380, Springer-Verlag 1997). Auch in diesem Fall ist das adsorbierte Enzym aktiv. Schließlich gibt es eine Vielzahl von Arbeiten, die sich damit beschäftigen, Urease für medizinische und andere Anwendungen zu immobilisieren, um gezielt Harnstoff in Ammoniak zu überführen. Anwendungen können hier unter anderem eine Herstellung von Sensoren zum Messen von Harnstoff im Blut sein. Weitere Träger, die hierzu für die Immobilisierung der Urease beschrieben sind, sind Ionentauscherharze vom Typ Amberlite MB-1 (A. Anita, C.A. Sastry, M.A. Hashim, Immobilization of urease using Amberlite MB-1, Bioprocess Engineering 17 (1997) 355-359, Springer-Verlag 1997), Textilien mit Ionenaustauschergruppen sowie unterschiedliche Cellulosen. Die aufgeführten Träger und hierzu zitierte Literatur zeigen, dass es gelingt, über unterschiedlichste Mechanismen Urease an unterschiedlichste feste Träger zu adsorbieren und dass die Urease im adsorbierten Zustand für eine Umsetzung von Harnstoff in Ammoniak aktiv ist. Deshalb ist es umso erstaunlicher, dass mit dem erfindungsgemäßen Konzept durch die Kombination von festen Trägern und Zinkionen und/oder Kupfer(II)-Ionen eine Inaktivierung der Urease erzielt werden kann.

Die auf der Oberfläche eines Trägers vorhandene Ladung lässt sich insbesondere durch eine Bestimmung des Zetapotentials charakterisieren. Das Zetapotential ist abhängig vom pH-Wert der Lösung, mit der die Grenzfläche des Festkörpers oder des Partikels in Kontakt steht, der Art des eingesetzten Elektrolyts, sowie der Konzentration des Elektrolyts. Experimentell lässt sich das Zetapotential beispielsweise durch elektrokinetische Messungen bestimmen. Eine häufig verwendete Methode ist die Elektrophorese, bei welcher die Wanderungsgeschwindigkeit der Teilchen im elektrischen Feld zur Bestimmung des Zetapotentials herangezogen wird. Die Meßmethode ist beispielsweise in G. Lagaly, O. Schulz, R. Zimehl; "Dispersionen und Emulsionen, eine Einführung in die Kolloidik fein verteilter Teilchen einschließlich der Tonminerale", Kapitel 10, "Oberflächenpotential und elektrokinetisches Potential", S. 347 ff., erschienen im Dr. Dietrich Steinkopff-Verlag, Darmstadt, 1997 beschrieben.

Es wurde überraschend als besonders vorteilhaft festgestellt und ist daher nach einer erfindungsgemäßen Ausführungsform bevorzugt, dass das Zetapotential des Trägers bei einem pH-Wert von 5 bis 9 beispielsweise weniger als 1 mV, bevorzugt weniger als -2,5 mV, besonders bevorzugt weniger als -5 mV beträgt. Insbesondere kann das Zetapotential des Trägers bei einem pH-Wert von 5,6, beispielsweise weniger als 1 mV, bevorzugt weniger als -2,5 mV, besonders bevorzugt weniger als -5 mV betragen.

Träger mit sehr guten Eigenschaften zur Verwendung im Rahmen der hierin beschriebenen Erfindung können nach einer bevorzugten Ausführungsform eine Kationenaustauschkapazität (CEC) von mehr als 10 meq pro 100g an Träger, vorzugsweise von mindestens 15 meq pro 100g an Träger, weiter bevorzugt von mindestens 25 meq pro 100g an Träger aufweisen.

Insbesondere können gemäß einer bevorzugten erfindungsgemäßen Ausführungsform als Träger Teilchen verwendet werden, die eine amorphe Phase und eine kristalline Phase umfassen. Derartige Teilchen weisen keine wohlgeordnete Struktur wie beispielsweise Bentonit oder Attapulgit auf, sondern umfassen neben einer kristallinen Phase, wie beispielsweise einer Smektit-Ton-Phase, eine amorphe Phase, beispielsweise eine amorphe Silica-Phase. Teilchen mit besonders guten Materialeigenschaften als Träger sind im makroskopischen Maßstab betrachtet homogen und stellen eine innige Mischung beider Phasen dar. Träger mit sehr guten Materialeigenschaften können als amorphe Phase eine Silica-Phase umfassen. Die amorphe Phase kann, wie nachstehend beschrieben, unter Verwendung einer Röntgenbeugungsanalyse, ermittelt werden. Zudem können Träger mit sehr guten Materialeigenschaften als kristalline Phase ein Schichtsilikat aufweisen. Träger mit besonders guten Materialeigenschaften weisen als kristalline Phase eine Smektit-Phase, insbesondere eine Montmorillonit-Phase, auf. Das Vorliegen einer Smektit-Phase, insbesondere eine Montmorillonit-Phase, kann beispielsweise durch den Methylenblau-Adsorptionstest erfasst werden, der nachstehend beschrieben ist. Im Allgemeinen kann die kristalline Phase unter Verwendung einer Röntgenbeugungsanalyse erfasst werden.

Die Existenz einer amorphen Silica-Phase neben einer Smektit-Phase lässt sich auch durch die chemische Analyse des entsprechenden Materials, insbesondere durch die Silikatanalyse, nachweisen. Im Falle eines solchen Materials findet man, selbst wenn man die $SiO_2$-Gehalte um ggf. vorhandenes $SiO_2$ aus kristallinen Silica-Phasen, wie z.B. Quarz oder Cristobalit, korrigiert, untypisch hohe $SiO_2$-Gehalte. Der $SiO_2$-Gehalt eines solchen Minerals, das Smektit und amorphes Silica umfasst oder aus diesem besteht, beträgt bevorzugt größer als 60 Gew.-%, besonders bevorzugt größer als 63 Gew.-%, ganz besonders bevorzugt größer als 66 Gew.-% $SiO_2$, bezogen auf das Gewicht des Minerals.

Gemäß einer möglichen Ausführungsform können die Teilchen als Silica-Mischphasen-Teilchen vorliegen. Derartige Teilchen können eine Phase, insbesondere eine kontinuierliche Phase, von amorphem Silica aufweisen, in der im Vergleich zur Silica-Phase räumlich kleinere, teilchenförmige Phasen, insbesondere Smektitphasen, vorliegen. Die Smektit-Phasen-Partikel können insbesondere in der kontinuierlichen Phase von amorphem Silica homogen verteilt und feststehend darin angeordnet sein. Insbesondere können die Teilchen ein Matrix-artiges Netzwerk von amorphem $SiO_2$ aufweisen, das Smektit-Phasen, die im Vergleich zu dem Volumen des Matrix-ähnlichen Netzwerks ein geringeres räumliches Volumen aufweisen, umfasst. Ohne dass die vorliegende Erfindung auf die Richtigkeit der nachstehenden Theorie beschränkt wäre, wird angenommen, dass Teilchen die eine Phase, insbesondere eine kontinuierliche Phase, von amorphem Silica aufweisen, in die im Vergleich zur Silica-Phase räumlich kleinere, teilchenförmige Smektitphasen eingefügt sind, sehr gute Materialeigenschaften für eine Verwendung als Träger im Rahmen der vorliegenden Erfindung aufweisen.

Die Struktur der Silica-Mischphasenteilchen kann durch verschiedene experimentelle Verfahren erfasst werden. Die aus amorphem Silica gebildete Matrix "verdünnt" die kristallinen Phasen, beispielsweise die Smektit-Phasen, insbesondere die Bentonit-Phasen. Dies kann zu einer Verringerung des "signalto-noise" ("Signal-Rausch")-Verhältnisses führen.

Weiterhin kann es, wenn die in der $SiO_2$-Matrix vorliegenden kristallinen Phasen in hohem Ausmaß delaminiert sind, zu einer starken Verbreiterung des entsprechenen Diffraktionspeaks kommen.

In einem XRD-Diffraktogramm der Silica-Mischphasen-Teilchen können die Reflexe sich nur gering über das Rauschen erheben. Das "signal-to-noise"-Verhältnis für Reflexe hinsichtlich der Teilchen, insbesondere hinsichtlich der Smektit-Phase, kann vorzugsweise in einem Bereich von 1 bis 1,2 sein. Verunreinigungen, wie beispielsweise Quarz, können jedoch zu scharfen Reflexen führen. Derartige Reflexe werden jedoch bei der Bestimmung des "signal-to-noise"-Verhältnisses nicht mit einbezogen. Vorzugsweise zeigen Silica-Smektit-Mischphasen-Teilchen, insbesondere Silica-Bentonit-Mischphasen-Teilchen keine oder nur eine geringe 001-Reflektion, welche den Schichtabstand in der Kristallstruktur beispielsweise der Bentonit-Teilchen angibt. Der Begriff "nur eine geringe Reflektion" gibt an, dass das "signal-to-noise"-Verhältnis der 001-Reflektion der Smektit-Phase beispielsweise weniger als 1,2 beträgt und vorzugsweise im Bereich 1,0 bis 1,1 liegt.

Eine quantitative Röntgenbeugung kann auf Basis des Rietveld-Verfeinerungs-Formalismus erfolgen. Einzelheiten des Verfahrens werden beispielsweise in R.A. Young: "The Rietveld Method", Oxford University Press, 1995 beschrieben. Hinsichtlich der Anwendung dieses Verfahrens auf die Analyse von Mineralproben wird beispielsweise auf D.K. McCarthy "Quantitative Mineral Analysis of Clay-bearing Mixtures", in: "The Reynolds Cup" Contest. IUCr CPD Newsletter, 27, 2002, 12 - 16 verwiesen. In der Praxis kann die quantitative Bestimmung der verschiedenen Mineralien in einer unbekannten Probe durch im Handel erhältliche Software, wie beispielsweise "Seifert AutoQuan" (erhältlich über Seifert/GE Inspection Technologies, Ahrensburg, Deutschland) erfolgen.

Die Menge an amorpher Silica-Phase und kristalliner Phase, beispielsweise Smektit-Phase, in Teilchen, insbesondere des Trägers, kann durch eine quantitative Röntgenbeugungsanalyse erfolgen. Einzelheiten eines derartigen Verfahrens sind in "Hand Book of Clay Science", F. Bergaya, B.K.G. Therry, G. Lagaly (Eds.), Elsevier, Oxford, Amsterdam, 2006, Kapitel 12.1: I. Srodon, Identification and Quantitative Analysis of Clay Minerals; "X-Ray Diffraction and the Identification and Analysis of Clay Minerals", D.M. Moora und R.C. Reaynolds, Oxford University Press, New York, 1997, S. 765 enthalten, wobei diese Schriften durch in Bezugnahme in die vorliegende Anmeldung aufgenommen werden.

Die Träger können in Form von Pulvern, Agglomeraten, Körnchen oder Granulaten vorliegen. Der teilchenförmige Träger kann eine beliebige von einem Fachmann ausgewählte Größe aufweisen. Insbesondere kann der Träger zu mindestens 80 Gew.-%, bezogen auf das Gesamtgewicht an Träger vor der Behandlung, insbesondere vollständig aus Teilchen mit einer größten Längenausdehnung von beispielsweise weniger als 5 mm, vorzugsweise von weniger als 1 mm, bevorzugt von weniger als 0,3 mm, insbesondere im Bereich von 0,2 $\mu$m bis 5 mm bestehen.

Es wird angenommen, dass neben der Auswahl der Träger im Hinblick auf eine gute Adsorption der Urease auch die Eigenschaft des Adsorptionsvermögens für Zinkionen und/oder Kupfer(II)-Ionen eine Rolle spielt. Im Hinblick auf diese Eigenschaft kann es nach einer erfindungsgemäßen Ausführungsform günstig sein, mit Trägern zu arbeiten, die Gruppen, insbesondere anionische Gruppen aufweisen, wie z.B. Kieselgel, smektitische Tonmineralien, Vermiculite, Ionenaustauschmaterialien, insbesondere Kationenaustauschmaterialien, Anionenaustauschmaterialien, amphotere Ionenaustauschmaterialien, insbesondere synthetische Anionenaustauschmaterialien oder anionische Cellulosen. Solche Träger können über elektrostatische Wechselwirkung (z.B. "Kationenaustausch") das $Zn^{2+}$ und/oder $Cu^{2+}$ an ihre Oberfläche binden.

Der Begriff "Ionenaustauschmaterialien" umfasst im Rahmen der vorliegenden Anmeldung sowohl feste Stoffe, als auch Fluide und Lösungen, die fähig sind positiv oder negativ geladene Ionen aus einer Wasser umfassenden Elektrolytlösung unter Abgabe äquivalenter Mengen anderer Ionen aufzunehmen. Gemäß der elektrischen Ladung der am Austausch beteiligten Ionen spricht man von "Kationenaustauschmaterialien" oder von "Anionenaustauschmaterialien". Ionenaustauschmaterialien, die mit beiden Ionenarten wechselwirken können, bezeichnet man als amphoter.

Träger, die im Rahmen der vorliegenden Erfindung sehr vorteilhafte Materialeigenschaften aufweisen, sind beispielsweise Träger, die ein oder mehrere Materialien, ausgewählt aus der Gruppe bestehend aus, $SiO_2$-haltigen Materialien, Titandioxiden, Aluminiumoxiden, Zinkoxiden, Zinksulfiden, Aktivkohlen, Holzkohlen, Zirkondioxiden, Polymeren, insbesondere anionisch geladene Polymere, Cellulosen, Dextrane und deren Gemischen umfassen oder hieraus bestehen. Anionisch geladene Polymere können auf Homo- oder Copolymeren basieren, die mindestens eine polymerisierbare Monomereinheit mit einer sauren Gruppe, insbesondere eine deprotonierte Carbonsäure- oder Sulfonsäuregruppe, umfassen.

Gemäß einer möglichen Ausführungsform können Kationenaustauscher oder Superabsorber oder Polymere mit anionischen (Seiten-)Gruppen, wie beispielsweise $-SO_4^-$ - Gruppen, $-SO_3^-$ - Gruppen,- $COO^-$ - Gruppen, etc., umfassende oder hieraus bestehende Träger mit $Zn^{2+}$ - und/oder $Cu^{2+}$ - Ionen behandelt werden, sowie Tonmaterialien umfassende oder hieraus bestehende Träger mit $Zn^{2+}$ - und/oder $Cu^{2+}$ - Ionen behandelt, insbesondere dotiert, werden.

Ohne dass die vorliegende Erfindung auf die Richtigkeit der nachstehenden Theorie beschränkt wäre, wird angenommen, dass ein fester teilchenförmiger Träger, der mit mindestens einer Zinkionen und/oder Kupfer(II)-Ionen enthaltenden Lösung vor dem Inkontaktbringen mit dem Urease enthaltenden fluiden Medium behandelt wurde, durch Austausch mit Alkali- und/oder Erdalkaliionen $Zn^{2+}$ - und/oder $Cu^{2+}$ - Ionen freisetzt. Bereits geringe Mengen an $Zn^{2+}$ - und/oder $Cu^{2+}$ reichen zur Inhibierung der Urease. Im Urin sind $Na^+$, $K^+$, $Ca^{2+}$, $Mg^{2+}$ in reichlichen Mengen vorhanden. Insbesondere kann die nachstehende Reaktion genutzt werden:

$$Polymer\text{-}A\text{-}Zn + Ca^{2+} \rightarrow Polymer\text{-}A\text{-}Ca + Zn^{2+},$$

(wie z.B.: $Polymer\text{-}SO_4\text{-}Zn + Ca^{2+} \rightarrow Polymer\text{-}SO_4\text{-}Ca + Zn^{2+}$),
wobei A eine anionische Seiten-(Gruppe) beschreibt und wobei beispielsweise $Polymer\text{-}A\text{-} = Polymer\text{-}SO_4\text{-}$, $Polymer\text{-}SO_3^-$, $Polymer\text{-}COO\text{-}$, $Polymer\text{-}O\text{-}$, etc. sein kann.

Träger, die geeignete Komplexbildungskonstanten aufweisen, und das Vorliegen von wirksamen Mengen an $Zn^{2+}$ - und/oder $Cu^{2+}$ - Ionen nach Behandlung des Trägers auf und /oder im Träger können durch einfache Reihenversuche ermittelt werden.

Optional kann ein Fachmann gemäß einer Ausführungsform keine anionisch geladenen Polymere, insbesondere keine Polymere, die eine deprotonierte Carbonsäure- oder Sulfonsäuregruppe aufweisen, als Träger einsetzen.

Gemäß einer erfindungsgemäßen Ausführungsform können die teilchenförmigen Träger auf ein Tragelement aufgebracht werden, das ein Material ausgewählt aus der Gruppe bestehend aus Aktivkohlen, Holzkohlen, Zirkondioxiden, Polymeren, insbesondere anionisch geladende Polymeren, Cellulosen, Dextrane und deren Gemischen umfasst oder hieraus besteht.

Bei dem $SiO_2$-haltigen Material kann es sich um mindestens ein $SiO_2$-haltiges Material handeln, das ausgewählt ist, aus der Gruppe, bestehend aus, Glasmaterialien, insbesondere porösen Glasmaterialien, Kieselsäuren, silikatische Materialien, Tonen und Gemischen hiervon.

Sehr vorteilhafte Materialeigenschaften weist beispielsweise ein Träger auf, worin es sich bei dem silikatischen Material um mindestens ein schichtsilikathaltiges Material, vorzugsweise mindestens ein phyllosilikathaltiges Material, um Glaspartikel, um Smektit-Silica-Mischphasen, insbesondere Bentonit-Silica-Mischphasen, umfassendes Material, um Zeolithe oder deren Gemische handelt.

Weiterhin kann das mindestens eine phyllosilikathaltige Material mindestens ein Phyllosilikat, ausgewählt aus der Gruppe bestehend aus Phyllosilikaten aus der Klasse der Smektite, Phyllosilikaten aus der Klasse der Vermiculite, Phyllosilikaten aus der Klasse der Glimmer und deren Gemischen umfassen oder hieraus bestehen.

Zudem kann es sich bei dem Smektit um einen Montmorillonit, insbesondere Bentonit, Vermiculit, Attapulgit, Saponit, Stevensit, Beidellit oder Gemische hiervon handeln.

Äußerst vorteilhafte Materialeigenschaften weist beispielsweise ein Träger auf, worin es sich bei dem silikatischen Material um eine oder mehrere Bleicherden handelt. Die eine oder die mehreren Bleicherden können vorzugsweise aus der Gruppe, bestehend aus Naturbleicherden, SMBE, HPBE und deren Gemischen ausgewählt werden. Insbesondere kann es sich bei der Naturbleicherde um eine Kieselgel und Smektit umfassende Mischung, vorzugsweise um eine natürlich vorkommende, Kieselgel und Smektit umfassende Mischung handeln.

Bei HPBE (High Performance Bleaching Earth) handelt es sich um eine Klasse von hochaktiven, meist auf Montmorillonit basierenden Bleicherden. Diese Klasse umfasst insbesondere säureaktivierte Montmorillonite, wobei die Säureaktivierung durch Dealuminieren der Rohtone mit konzentrierten Säuren bei hohen Temperaturen, meist bei Siedehitze, durchgeführt wird. Bei diesem Verfahren wird ein Bleicherdeprodukt mit sehr großer spezifischer Oberfläche und großem Porenvolumen erhalten. Naturbleicherden, die auch als natürlich vorkommende Bleicherden oder NABE (Natural Active Bleaching Earth) bezeichnet werden, bilden eine weitere Klasse. Eine dritte Bleicherdeklasse bilden so genannte oberflächenaktivierte Systeme (SMBE = Surface Modified Bleaching Earth; oberflächenaktivierte Bleicherden). Zu deren Herstellung wird ein naturaktiver Rohton mit geringen Säuremengen beaufschlagt und somit eine "in situ Aktivierung" erreicht. Für dieses Verfahren haben sich insbesondere Attapulgit und Hormit enthaltende Rohtone bewährt.

Die vorstehend genannten SiO$_2$-haltigen Materialien sind einem Fachmann bekannt. Silikate, sowie ihr Aufbau und ihre Zusammensetzung werden zudem beispielsweise in dem Lehrbuch "Holleman-Wiberg, Lehrbuch der Anorganischen Chemie" von N. Wiberg, 91.-100. Aufl., Walter de Gruyter & Co., 1985, ISBN 3-11-007511-3, S. 768 bis 779 erläutert.

Die Behandlung des festen teilchenförmigen Trägermaterials mit der mindestens einen Zinkionen und/oder Kupfer(II)-Ionen enthaltenden Lösung kann durch ein dem Fachmann bekanntes Verfahren erfolgen. Träger mit besonders guten Inhibierungseigenschaften können beispielsweise durch Aufsprühen, Tränken oder Suspendieren des festen teilchenförmigen Trägermaterials mit und/oder in der mindestens einen Zinkionen und/oder Kupfer(II)-Ionen enthaltenden Lösung erfolgen.

Gemäß einer weiteren Ausführungsform kann ein Träger, insbesondere ein quellfähiger Träger, beispielsweise ein Superabsorber, der optional keine Carboxylgruppen aufweist, in eine Zinkionen und/oder Kupfer(II)-Ionen enthaltende Lösung eingebracht werden, insbesondere dort quellen gelassen werden. Nach einem anschließenden teilweisen oder vollständigen Trocknen werden optional überschüssige Zinkionen und/oder Kupfer(II)-Ionen ausgewaschen, beispielsweise mit Wasser. Vorzugsweise kann anschließend eine teilweise oder vollständige Trocknung des gewaschenen Trägers erfolgen. Der Träger kann nach einer möglichen Ausführungsform anschließend mit Absorbentien, insbesondere Superabsorbern, die keine Behandlung mit einer Zinkionen und/oder Kupfer(II)-Ionen enthaltenden Lösung erfahren haben, vermischt oder diesen, beispielsweise abschnitts- oder schichtweise, beigefügt werden. Die Mengenverhältnisse an Absorbentien, insbesondere Superabsorbern, und an behandeltem Träger werden von einem Fachmann derart gewählt, dass Zinkionen und/oder Kupfer(II)-Ionen in ausreichender Menge freigesetzt werden, um die Urease zu inhibieren.

Bei den festen Trägermaterialien kann es sich auch um solche handeln, bei denen ein Trägerkern durch funktionale Gruppen modifiziert wurde. Bei der Oberflächenmodifikation kann es sich beispielsweise um eine Silanisierung, um eine Beaufschlagung mit einem Polymer oder mit einer sogenannten Solgelbeschichtung handeln. Dies schließt Systeme ein, bei denen beispielsweise Fasern oder Windelfliese mit anorganischen Materialien über eine Solgelbeschichtung modifiziert wurden, d.h. durch eine Behandlung mit einer kolloidalen Lösung einem reaktiven Metallalkoxid oder einer Mischung aus beiden Systemen, die ggf. auch noch organische Gruppen enthalten können.

Der behandelte Träger kann vor einem optionalen Aufbringen auf und/oder Einbringen in ein Absorbens, insbesondere Superabsorber und/oder vor dem in Kontakt kommen mit dem fluiden Medium insbesondere in trockener Form, als Aufschlämmung oder als Suspension vorliegen. Gemäß einer Ausführungsform kann der feste, teilchenförmige Träger auf eine Trägereinrichtung, beispielsweise ein Blatt oder Streifen, insbesondere aus einem Vlies- oder Netz-Material, beispielsweise aus Polymermaterial und/oder Absorbensmaterial aufgebracht werden und dann mit der mindestens einen Zinkionen und/oder Kupfer(II)-Ionen umfassenden Lösung auf eine dem Fachmann bekannte Weise, beispielsweise durch Aufsprühen, behandelt werden. Diese Trägereinrichtung kann dann über, unter oder zwischen Lagen, die Absorbens, insbesondere Superabsorber, umfassen oder hieraus bestehen, angeordnet werden.

Die Verwendung eines festen, teilchenförmigen Trägers zur Inhibierung der Aktivität von Urease in einem Urease enthaltenden fluiden Medium kann insbesondere in Gegenwart eines Absorbens und/oder Superabsorbers erfolgen. Insbesondere kann die Verwendung in einem Produkt erfolgen, das mindestens ein Absorbens enthält, wobei das mindestens eine Absorbens vorzugsweise aus mindestens einem Superabsorber besteht oder diesen umfasst.

Besonders gute Ergebnisse konnten bei der erfindungsgemäßen Verwendung erzielt werden, wenn die Verwendung in einem Produkt erfolgt, das mindestens einen Superabsorber umfasst. Vorzugsweise kann der mindestens eine Superabsorber ein Polymer umfassen oder aus diesem bestehen, das ausgewählt ist, aus der Gruppe bestehend aus Polyacrylsäuren, Polyacrylaten, Carboxy-Cellulosen, insbesondere Carboxymethylcellulosen, und deren Gemischen.

Als Absorbens können dem Fachmann bekannte Flüssigkeiten absorbierende Materialien oder Materialiengemische verwendet werden, die ein Fachmann auf Basis seines allgemeinen Fachwissens und der Lehre der vorliegenden Anmeldung auswählen kann. Beispiele für Absorbentien sind unter anderem Baumwollfasern, absorbierende Gelmaterialien, zerkleinerter Holzzellstoff, Fluffzellstoff, mit einem Schmelz-Blas-Verfahren hergestellte Polymere, Zellulosewatte, insbesondere gekreppte Zellulosewatte, Zellulosefasern, insbesondere chemisch modifizierte und/oder quervernetzte Zellulosefasern, Gewebe, einschließlich Gewebelaminate, absorbierende Schäume, absorbierende Schwämme, Superabsorber, sowie Gemische dieser Absorbentien.

Vorzugsweise umfasst das Absorbens von 11 bis 75 Gew.-%, bevorzugt von 21 bis 67 Gew.-% an Superabsorber, bezogen auf das Gesamtgewicht an Absorbens. Bei einem Superabsorber kann es sich sowohl um einen sauren, als auch einen nicht-sauren Superabsorber oder deren Gemische handeln.

Gute Ergebnisse können insbesondere erhalten werden, wenn das Absorbens mindestens einen Superabsorber umfasst, wobei es sich bei dem Superabsorber um einen sauren oder einen nicht-sauren Superabsorber handeln kann. Die Funktionsweise von Superabsorbern kann insbesondere darauf basieren, dass ein Superabsorber bei Kontakt mit Wasser, Blut und/oder Urin, etc. ein Hydrogel ausbildet.

Vorzugsweise kann das Gewichtsverhältnis Träger zu Absorbens beispielsweise 1:3 bis 1:30, vorzugsweise 1:5 bis 1:15, bevorzugt 1:8 bis 1:12 betragen. Weiterhin kann beispielsweise das Gewichtsverhältnis Träger zu Superabsorber 1:4 bis 1:99, vorzugsweise 1:5 bis 1:50, bevorzugt 1:8 bis 1:25 betragen.

Der Begriff "Superabsorber" ist einem Fachmann bekannt und bezeichnet in Wasser quellfähige, wasserunlösliche Materialien, die ein Mehrfaches ihres eigenen Gewichts an Wasser und/oder Körperfluiden aufnehmen können. Vorzugsweise kann ein Superabsorber mindestens etwa das Zehnfache seines Gewichts, bevorzugt mindestens etwa das Achtzehnfache seines Gewichts an einer wässrigen 0,9 Gew.-% Natriumchlorid enthaltenden Lösung aufnehmen. Eine Ermittlung der Absorptionsfähigkeit des Superabsorbens kann vorzugsweise nach dem Standard-Testverfahren EDANA WSP 241.2 erfolgen.

Superabsorber können beispielsweise aus sauren Superabsorber-Polymeren bestehen oder diese umfassen. Saure Superabsorber-Polymere können auf Homo- oder Copolymeren basieren, die mindestens eine polymerisierbare Monomereinheit mit einer sauren Gruppe, insbesondere eine Carbonsäure- oder Sulfonsäuregruppe, umfassen. Beispiele hierfür sind Methacrylsäure, Acrylsäure oder Vinylsulfonsäure. Beispiele geeigneter Polymere sind u.a. Poly(meth)acrylsäuren, Polymere und Copolymere von Vinylsulfonsäuren, Polyacrylate und Acrylsäure-Stärke-Pfropfpolymere. Vorzugsweise sind Superabsorber-Polymere quervernetzt, um die Polymere weitgehend oder vollständig wasserunlöslich zu machen. Eine Herstellung eines Superabsorbers wird beispielsweise in der EP 0 391 108 A2 beschrieben, wobei die Offenbarung dieser Schrift durch Inbezugnahme in die vorliegende Anmeldung aufgenommen wird.

Der saure Superabsorber kann beispielsweise einen pH-Wert von 3,0 bis 5,5, vorzugsweise von 3,8 bis 5,2 aufweisen. Eine Messung des pH-Werts kann vorzugsweise nach dem Standard-Testverfahren EDANA WSP 200.2 erfolgen.

Das Absorbens, insbesondere der Superabsorber, kann beispielsweise in Form von Partikeln, Kügelchen, Fasern, Flocken, usw. vorliegen.

Die erfindungsgemäße Verwendung eines festen, teilchenförmigen Trägers zur Inhibierung der Aktivität von Urease, in einem Urease enthaltenden fluiden Medium kann insbesondere zur Verhinderung oder Verringerung einer Geruchsbelastung, insbesondere eines Geruchs von Ammoniak, aus urinhaltigen fluiden Medien, insbesondere Urin oder urinhaltigen Gemischen, eingesetzt werden.

Insbesondere kann die erfindungsgemäße Verwendung eines festen, teilchenförmigen Trägers zur Inhibierung der Aktivität von Urease in einem absorbierenden Artikel, wie beispielsweise, jedoch nicht ausschließlich einer Windel, Windeleinlage, Binde, insbesondere für eine Verwendung in Unterwäsche, Einlage, Betteinlage, einem Tuch, einem Inkontinenzartikel, beispielsweise in Form einer Hygieneeinlage oder Hygienebinde, sowie in Haustierstreu erfolgen. Zudem kann die erfindungsgemäße Verwendung eines festen, teilchenförmigen Trägers zur Inhibierung der Aktivität von Urease zur Verhinderung oder Verringerung einer Geruchsentwicklung, insbesondere einer Ammoniak-Geruchsentwicklung in einem absorbierenden Artikel nach in Kontakt kommen mit einem urinhaltigen fluiden Medium eingesetzt werden. Die absorbierenden Artikel sind nicht auf absorbierende Artikel beschränkt, die für eine Urinaufnahme ausgelegt oder vorgesehen sind, sondern umfassen auch absorbierende Artikel, die für eine Aufnahme von Blut, insbesondere Menstruationsfluid, und beliebige Körperausscheidungen, einschließlich Schweiß, vorgesehen oder ausgelegt sind.

Haustierstreu, insbesondere Katzenstreu, umfasst üblicherweise ein festes Absorbens. Bei dem festen Absorbens kann es sich beispielsweise um Si-haltige Mineralien, wie beispielsweise Montmorillonite, Bentonite, Kaolinite, sowie um Sägemehl, absorbierende Fasermaterialien, Flugasche, Kunststoffschäume und deren Gemische handeln. Eine erfindungsgemäße Ureaseinhibitor-Träger-Zusammensetzung kann in einer Menge von beispielsweise 0,01 bis 80 Gew.-%, bezogen auf des Gesamtgewicht des Haustierstreus, in Haustierstreu enthalten sein.

Weiterhin kann die erfindungsgemäße Verwendung eines festen, teilchenförmigen Trägers zur Inhibierung der Aktivität oder Inaktivierung von Urease in Tierställen, Katzenklos oder Tierkäfigen (wobei der Begriff Tierkäfig auch Tiertransportbehälter und jegliche Tiergehege- und/oder Tieraufbewahrungseinrichtungen umfasst) und/oder zur Verhinderung oder Verringerung eines Abbaus von Harnstoff basiertem Dünger durch Reaktion mit Urease erfolgen.

Gemäß einem weiteren Aspekt stellt die vorliegende Erfindung ein Verfahren zur Herstellung einer Ureaseinhibitor-Zusammensetzung bereit. Dieses Verfahren umfasst zunächst das Bereitstellen mindestens eines festen teilchenförmigen Trägers, sowie das Bereitstellen mindestens einer Zinkionen und/oder Kupfer(II)-Ionen enthaltenden Lösung. Anschließend erfolgt ein Behandeln des mindestens einen festen teilchenförmigen Trägers mit der mindestens einen Zinkionen und/oder Kupfer(II)-Ionen enthaltenden Lösung, vorzugsweise in Abwesenheit eines Urease enthaltenden fluiden Mediums. Anschließend erfolgt optional ein Trocknen und/oder optional ein Waschen (z.B. mit Wasser) des mit der Zinkionen und/oder Kupfer(II)-Ionen enthaltenden Lösung behandelten, festen teilchenförmigen Trägers. Der mit der Zinkionen und/oder Kupfer(II)-Ionen enthaltenden Lösung behandelte Träger kann dann optional mit einem Absorbens, insbesondere einem Superabsorber, auf eine dem Fachmann bekannte Weise in Kontakt gebracht werden, insbesondere auf diesen aufgebracht und/oder in diesen eingebracht werden.

Das Trocknen kann beispielsweise bei einer Temperatur im Bereich von mindestens 30˚C, vorzugsweise von mindestens 70˚C, insbesondere bei einer Temperatur im Bereich von 30-180˚C, auch bei einem gegenüber dem Umgebungsdruck verringerten Druck, beispielsweise von unter 1000 hPa, erfolgen.

Gemäß einem weiteren Aspekt betrifft die vorliegende Erfindung einen absorbierenden Artikel, insbesondere Windel, Windeleinlage, Binde, insbesondere für eine Verwendung in Unterwäsche, Einlage, insbesondere für eine Verwendung in Unterwäsche, Tuch, Betteinlage, Inkontinenzartikel, beispielsweise in Form einer Hygieneeinlage oder Hygienebinde, sowie Haustierstreu, der mindestens ein Absorbens, insbesondere mindestens einen Superabsorber, sowie einen mit mindestens einer Zinkionen und/oder Kupfer(II)-Ionen enthaltenden Lösung behandelten festen teilchenförmigen Träger umfasst. Vorzugsweise kann der Superabsorber aus der Gruppe, bestehend aus Polyacrylaten, Polyacrylsäuren, Carboxy-Cellulosen, insbesondere Carboxymethylcellulosen, und deren Gemischen ausgewählt sein.

Eine erfindungsgemäße Verwendung eines mit Zinkionen und/oder Kupfer(II)-Ionen behandelten Trägers führt auch in Gegenwart eines Carboxylgruppen aufweisenden Superabsorbers überraschenderweise zu sehr guten Ergebnissen bei einer Inhibierung von Urease. Ohne dass die vorliegende Erfindung auf die Richtigkeit der nachstehenden Theorie beschränkt wäre, wird angenommen, dass Superabsorber, die beispielsweise in signifikanten Mengen Kationen komplexierende Gruppen aufweisen können, Zinkionen und Kupfer(II)-Ionen gut komplexieren oder durch weitere chemische und/oder physikalische Wechselwirkungen abfangen können. Dies trifft insbesondere zu, wenn der Superabsorber ein Superabsorber ist, der Polyacrylsäuren und/oder deren Salze umfasst. Dies hat zur Folge, dass ein mit Zinksalz- und/oder Kupfer(II)-Salzlösungen in Kontakt gebrachter, Carboxylgruppen aufweisender Superabsorber nicht oder nur für eine kurze Zeitspanne Urease inhibiert (im Vergleich zu einer Verwendung eines mit Zinkionen und/oder Kupfer(II)-Ionen behandelten Trägers). Ohne dass die vorliegende Erfindung auf die Richtigkeit der nachstehenden Theorie beschränkt wäre, wird angenommen, dass Zinkionen und/oder Kupfer(II)-Ionen in Gegenwart eines Carboxylgruppen aufweisenden Superabsorbers zu einem vergleichsweise geringen Teil in die aktive Tasche der Urease wandern und dort gebunden werden können und überwiegend zum Superabsorber diffundieren, da der Superabsorber eine hohe Konzentration an komplexierenden Gruppen und/oder Zinkionen und/oder Kupfer(II)-Ionen abfangenden Gruppen aufweist.

Ein absorbierender Artikel kann weiterhin optional eine oder mehrere Decklagen und/oder eine oder mehrere Bodenlagen aufweisen. Als Decklage wird eine Lage bezeichnet, welche das Absorbens teilweise oder vollständig bedeckt und durch die vorzugsweise das zu absorbierende Fluid, beispielsweise der Urin, zunächst durchtritt, bevor es mit dem Absorbens in Kontakt kommt. Geeignete Decklagen können durch einen Fachmann anwendungsspezifisch ausgewählt werden. Beispielsweise können gewobene Materialien oder nicht-gewobene Materialien, wie beispielsweise Vliesmaterialien oder Fasergelege, Polymermaterialien, wie beispielsweise poröse oder netzartige Schäume, mit Öffnungen versehene Kunststoffbahnen, netzartige thermoplastische Schichten mit besonders guten Ergebnissen als Decklage verwendet werden. Decklagen können beispielsweise natürliche Fasern, beispielsweise Holz- oder Baumwollfasern und/oder synthetische Fasern, beispielsweise Polyesterfasern, Polyethylenfasern, Polypropylenfasern, oder Gemische dieser Fasern umfassen.

Optional können zwischen und/oder auf und/oder unter dem einen oder mehreren Absorbern eine oder mehrere weitere Schichten angeordnet sein, die beispielsweise Schaummaterialen, Faservliese oder Gewebe umfassen können.

Optional kann der absorbierende Artikel zudem noch eine oder mehrere Bodenlagen aufweisen, die vorzugsweise eine oder mehrere Seiten des mindestens einen Absorbers oder der Anordnung des mindestens einen Absorbers und der einen oder mehreren weiteren Decklagen und optionalen weiteren Schichten, zumindest teilweise umhüllen. Vorzugsweise kann eine Bodenlage wasserundurchlässiges Material, beispielsweise eine wasserundurchlässige Kunststofffolie umfassen oder hieraus bestehen.

**Methodenteil**

Zur Bestimmung der Parameter des Gegenstands der vorliegenden Erfindung werden die nachstehenden Methoden eingesetzt:

a) Bestimmung der Löslichkeit

Eine Bestimmung der Löslichkeit eines Salzes kann erfolgen, indem bei einer Temperatur von 25 ˚C und einem Druck von 100 kPa eine gesättigte Lösung eines Salzes hergestellt wird und das Volumen der gesättigten Lösung des Salzes, sowie die Masse an Salz (und damit die Menge an Salz) in der gesättigten Lösung des Salzes, bezogen auf das Volumen der gesättigten Lösung des Salzes, bei einer Temperatur von 25 ˚C und einem Druck von 100 kPa ermittelt wird. Die Bestimmung des Volumens kann durch eine Verwendung eines graduierten Gefäßes erfolgen.

b) Bestimmung der Ammoniakbildung

Die Ammoniakkonzentration lässt sich insbesondere mit einer ammoniaksensitiven Elektrode elektrochemisch bestimmen, beispielsweise unter Verwendung der Elektrode von Thermo "Orion 95-12 Ammonia-Electrode" (Sensortechnik Meinsberg GmbH, Kurt-Schwabe-Straße 6, OT Meinsberg, 04720 Ziegra-Knobelsdorf).

c) Spezifische Oberfläche/Porenvolumen

Die Bestimmung erfolgt nach der BET-Methode gemäß DIN 66131; eine Veröffentlichung der BET-Methode findet sich auch in J. Am. Chem. Soc. 60, 309 (1938).

Das Porenvolumen wurde unter Anwendung der BJH Methode ermittelt (E.P. Barrett, L.G. Joyner, P.P. Hienda, J. Am. Chem. Soc. 73 (1951) 373). Porenvolumina bestimmter Porengrössenbereiche werden durch Aufsummieren inkrementeller Porenvolumina bestimmt, die aus der Auswertung der Adsorptionsisotherme nach BJH erhalten werden.

d) Glühverlust:

Der Glühverlust kann nach DIN ISO 803/806 bestimmt werden.

e) Bestimmung des Zetapotentials:

Das Zetapotential wurde anhand der Wanderungsgeschwindigkeit im elektrischen Feld bestimmt. Hierzu wurde ein Zetasizer Nano Series der Fa. Malvern Instruments Ltd. (Malvern, Worcestershire, WR14 1XZ, England) eingesetzt. Die Messeinstellungen sind wie folgt:

Zugrundegelegtes Messmodell

| | |
|---|---|
| (General Options): | Smoluchowski |
| Zugrundegelegtes Auswertemodell: | General Purpose |
| Messtemperatur: | 25 ˚C |

Es wurde ein Feststoffgehalt (Trägergehalt) von 0,1 Gew % eingestellt, bezogen auf das Gewicht des zur Messung verwendeten Puffers.

Die Herstellung der Messprobe zur Erfassung des Zetapotentials kann insbesondere wie folgt durchgeführt werden. Der Träger wird einer Vermahlung auf eine mittlere Teilchengröße von 8 $\mu$m unterzogen. Die so erhaltenen Pulver werden in einer Konzentrationen von 0,1 Gew.-% in einem Puffer dispergiert (z. B. in Acetat-Puffer (50 mmol/L, pH 5,6 $\pm$ 0,25).

f) Röntgendiffraktometrie:

1 bis 2 g einer Probe wurden trocken per Hand in einer Achat-Reibschale vermahlen und durch ein 20 μm Sieb gesiebt. Dieser Vorgang wird solange wiederholt bis die vollständige Probenmenge durch das Sieb durchtrat.

Die Röntgenbeugungsaufnahmen werden an dem Gerät Siemens D5000 erstellt. Die folgenden Bedingungen wurden verwendet:

| | |
|---|---|
| Probenhalter | Kunststoff, "top loading", $\varnothing$ = 25 mm |
| Dicke der Pulverschicht | 1 mm |
| Röntgenröhre | Cu K$\alpha$: 40 kV/40mA |
| Diffraktionswinkel | 2 - 80 ˚ (2 θ) |
| Messzeit | 3 Sekunden pro Stufe |
| Spalte | Primäre und sekundäre Divergenzspalte von 1 mm |

Eine qualitative Auswertung der Diffraktogramme (Zuweisung der Mineralphase erfolgte mit dem Computerprogramm "EVA" von Bruker AXS GmbH, Karlsruhe und gemäß der Veröffentlichung von Brindley & Brown (1980): Crystal structures of clay minerals and their x-ray identification. - Mineralogical Society No. 5, 495.

Die quantitative Auswertung erfolgte gemäß dem Rietveld Verfahren unter Verwendung des Computerprogramms AutoQuan von Seifert (GE Inspection Technologies, Ahrensburg, Deutschland) auf Basis des Rietveld Verfahrens (siehe Beschreibung) zur Bestimmung des Gehalts an Röntgen-amorphen Materialien wurde Zincit als interner Standard zugegeben. Zur Korrektur des Hintergrunds wurde ein Polynom vierter Ordnung in dem Winkelbereich 4 - 80 ˚ in 2 θ verwendet.

g) Gehaltsbestimmung über die Methylenblauadsorption

Herstellung einer Tetranatriumdiphosphat-Lösung

5,41 g Tetranatriumdiphosphat werden auf 0,001 g genau in einen 1000 ml Messkolben eingewogen und unter Schütteln bis zur Eichmarke mit dest. Wasser aufgefüllt.

Herstellung einer 0,5 %-igen Methylenblaulösung

In einem 2000 ml Becherglas werden 125 g Methylenblau in 1500 ml dest. Wasser gelöst. Die Lösung wird abdekantiert und auf 25 l mit dest. Wasser aufgefüllt.

0,5 g feuchter Testbentonit mit bekannter innerer Oberfläche werden in einem Erlenmeyerkolben auf 0,001 g genau eingewogen. Es werden 50 ml Tetranatriumdiphosphatlösung zugegeben und die Mischung 5 Minuten zum Sieden erhitzt. Nach dem Abkühlen auf Raumtemperatur werden 10 ml 0,5 molare $H_2SO_4$ zugegeben und 80 bis 95 % des zu erwartenden Endverbrauchs an Methylenblaulösung zugegeben. Mit dem Glasstab wird ein Tropfen der Suspension aufgenommen und auf ein Filterpapier gegeben. Es bildet sich ein blau-schwarzer Fleck mit einem farblosen Hof. Es wird nun in Portionen von 1 ml weitere Methylenblaulösung zugegeben und die Tüpfelprobe wiederholt. Die Zugabe erfolgt solange, bis sich der Hof leicht hellblau färbt, also die zugegebene Methylenblaumenge nicht mehr vom Testbentonit absorbiert wird.

Prüfung von Tonmaterialien

Die Prüfung des Tonmaterials wird in der gleichen Weise durchgeführt wie für den Testbentonit. Aus der verbrauchten Menge an Methylenblaulösung lässt sich die innere Oberfläche des Tonmaterials berechnen.

381 mg Methylenblau/g Ton entsprechen nach diesem Verfahren einem Gehalt von 100 % Montmorillonit.

h) Prinzip der CEC-Bestimmung:

Der zu untersuchende Träger, beispielsweise ein Schichtsilikat enthaltendes oder aus Schichtsilikat bestehendes Material, beispielsweise ein Ton, wird mit einem großen Überschuss an wässriger $NH_4Cl$ - Lösung behandelt, ausgewaschen und die auf dem Träger verbliebene $Nah_4^+$ - Menge als Stickstoff an einem Elementaranalysengerät (Fa. Elementar) bestimmt.

$$Me^+ (Ton)^- + NH_4^+ \rightarrow NH_4^+(Ton)^- + Me^+$$

$$(Me^+ = K^+, Na^+, 1/2 \, C_a^{2+}, 1/2 \, Mg^{2+}....)$$

Geräte: Sieb 63 $\mu$m; Erlenmeyer - Schliffkolben, 300ml; Analysenwaage; Membranfilternutsche, 400 ml; Zellulose-Nitrat-Filter, 0,2$\mu$m - ( Fa. Machery & Nagel ); Vakuumsaugflasche 500ml; Trockenschrank 110 ˚C; Rückflusskühler; Heizplatte; 250ml Messkolben; 600 ml Becherglas; ICP - OES; Pipette 5 ml; 25 ml Messzylinder.

Chemikalien: $NH_4Cl$ - Lösung, 2mol/l p.a. (Merck); Salzsäure 1:2.

Durchführung: 5 g Träger werden quantitativ durch ein 63 $\mu$m Sieb gesiebt und bei 110 ˚C bis zur Gewichtskonstanz getrocknet. Danach werden auf der Analysenwaage zwischen 200 und 300 mg in einen Erlenmeyer-Schliffkolben eingewogen. Man gibt 25 ml 2 molare $NH_4Cl$ - Lösung zu und kocht die Suspension eine Stunde am Rückfluss.

Nach einer Standzeit von 24 h wird der $NH_4^+$ - behandelte Träger über eine Membranfilternutsche abfiltriert. Mit destilliertem Wasser wird der Filterkuchen in kleinen Portionen mit insgesamt 1200 ml Wasser gewaschen, das anschließend portionsweise in einem 600 ml Becherglas eingeengt wird.

Der ausgewaschene $NH_4^+$ - behandelte Träger wird vom Filter abgenommen und während 16 Stunden bei 110 ˚C getrocknet. Vom trockenen Filterkuchen wird der N-Gehalt am Elementaranalysator bestimmt.

Berechnung der Gesamtkationenaustauschfähigkeit:

Die Gesamtkationenaustauschfähigkeit (CEC) des Trägers ist der mittels Elementaranalyse ermittelte $NH_4^+$-Gehalt des $NH_4^+$-behandelten Trägers. Die Angaben erfolgen in mval/100 g Träger (meq/100g). Hierbei wird angenommen, dass aller Stickstoff in Form von $NH_4^+$ vorliegt.

Beispiel:

Stickstoff-Gehalt = 0,93 %;
Molekulargewicht: N = 14,0067 g/mol

$$CEC = \frac{0,93 \times 1000}{14,0067} = 66,4 \text{ mVal/100g Träger}$$

Gewichtsangaben beziehen sich, wenn nicht explizit abweichend angegeben, auf trockenes Material, das nach Trocknung bei 90˚C bis zur Gewichtskonstanz erhalten wurde. Im Falle von Superabsorbern wird als trockener Superabsorber ein Superabsorber angesehen, zu dem kein Wasser zugefügt wurde und der lediglich unvermeidliches, vom Herstellungsverfahren herrührendes Restwasser umfasst.

Die Erfindung wird nun anhand der nachstehenden, nicht beschränkenden Beispiele näher erläutert:

BEISPIELE

Beispiel 1:

Versuchsreihe 1: Urease-Inhibierung durch mit Zinkionen behandelte Träger in Gegenwart eines Superabsorbers

a) Behandlung des Trägers

In einem 20 ml Becherglas werden 2 g Träger eingewogen. Anschließend werden 10 ml Wasser und 2 ml einer 20 Gew.%-igen Zinkacetat-Lösung zugegeben. Die Träger werden bei 120˚C getrocknet und anschließend in einen Faltenfilter eingefüllt und jeweils mit 800 ml Wasser gewaschen. Daraufhin erfolgt eine Trocknung im Trockenschrank bei 70˚C.

b) Messung

Die nachstehend angegebene Menge an mit Zinkacetat behandeltem Träger, der gemäß Beispiel 1a hergestellt wurde, sowie 0,1 g Superabsorber (Polyacrylsäure zur Verwendung in Windeln) werden in 40 ml 2 Gew.%-iger Harnstoff-Lösung eingewogen. Anschließend werden sofort 2 ml einer 2 Gew.%-igen Urease-Lösung (Urease von Carl-Roth, etwa 1 U/mg, f.d. Biochemie, Art.-Nr.: 7573.1) zugegeben und der Spannungsverlauf bei einer Messung mit dem nachstehend angegebenen Messsystem notiert und hieraus die Konzentration an Ammoniak in der Lösung ermittelt. Das Messystem war ein Mehrparameter Messsystem KM2000 von Sensortechnik Meinsberg (Kurt-Schwabe-Straße 6, OT Meinsberg, 04720 Ziegra-Knobelsdorf). Daran angeschlossen war eine EGA 133 pH-Elektrode sowie eine Orion 95-12 Ammonia-Elektrode von Thermo Scientific.

Zu Vergleichszwecken wurden zudem identisch durchgeführte Messungen vorgenommen, bei denen jedoch anstelle des mit Zinkionen behandelten Trägers die nachstehend jeweils angegebene Menge an unbehandeltem Träger oder an Zinkacetat zugegeben wurde.

c) Zinkacetat belegtes Zinkoxid

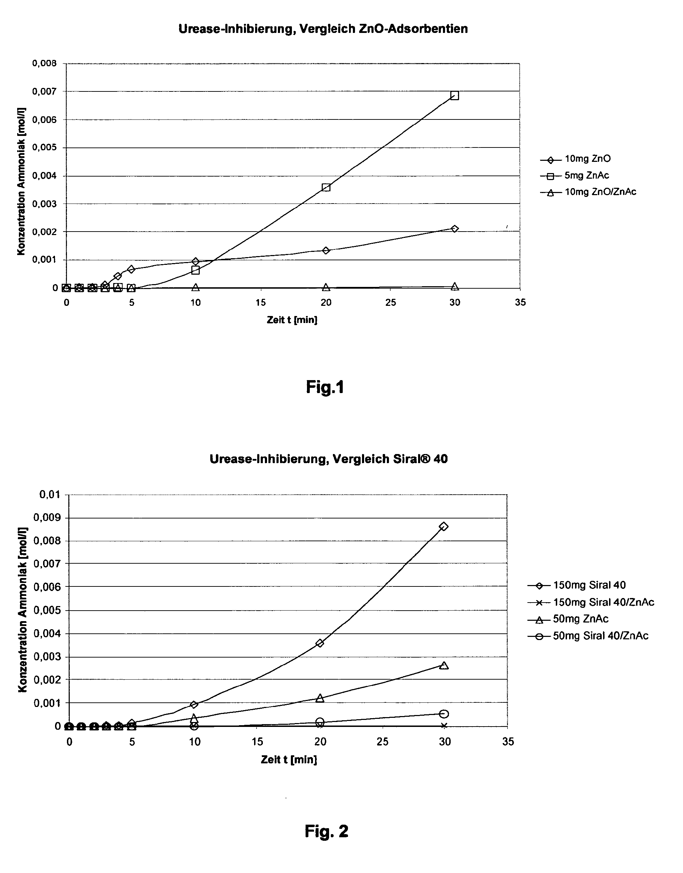

**Fig. 1** zeigt die Urease-Inhibierung in Gegenwart von 10 mg mit Zinkacetat belegtem Zinkoxid bei einer gemäß Beispiel 1b durchgeführten Messung. Zu Vergleichszwecken wurde zudem die Urease-Inhibierung in Gegenwart von 10 mg Zinkoxid (ZnO," >99% reinst." von Carl-Roth Art.-Nr.: 9348.1) beziehungsweise 5 mg Zinkacetat ermittelt. Deutlich erkennbar ist hierbei die Inhibierung von Urease durch mit Zinkacetat belegtes Zinkoxid. Sogar bei einer Zugabe von 5 mg reinem Zinkacetat entsteht während der Vergleichsmessung eine größere Menge an Ammoniak, obwohl in diesem Fall eine deutlich höhere Menge an freien Zinkionen vorliegt.

d) Zinkacetat belegtes Siral® 40

**Fig. 2** zeigt die Urease-Inhibierung in Gegenwart von 50 mg (sowie 150 mg) mit Zinkacetat belegtem Siral® 40 (Sasol Alumina; Sasol Germany, Anckelmannsplatz 1, 20537 Hamburg) bei einer gemäß Beispiel 1b durchgeführten Messung. Bei Siral® 40 handelt es sich um ein Mischoxid aus $SiO_2$ und $Al_2O_3$ mit einem Anteil von 40 Gew.-% $SiO_2$. Die charakteristischen Daten sind in der folgenden Tabelle aufgeführt:

**Tabelle 1:**

| Chemische und physikalische Eigenschaften von Siral® 40: | |
|---|---|
| Zusammensetzung | Siral® 40 |
| $Al_2O_3$-Gehalt (Gew.-%, bezogen auf das Gesamtgewicht an Siral® 40) | 75 |
| LOI (%) [*)] | 25 |
| $Al_2O_3$ : $SiO_2$ (w) / (w) | 60:40 |
| C (Gew.-%, bezogen auf das Gesamtgewicht an Siral® 40) | 0.2 |
| $Fe_2O_3$ (Gew.-%, bezogen auf das Gesamtgewicht an Siral® 40) | 0.02 |
| $Na_2O$ (Gew.-%, bezogen auf das Gesamtgewicht an SIral® 40) | 0.005 |
| Eigenschaften: | |
| Schüttgewicht(g/l) | 250-450 |
| Mittlere Teilchengröße ($d_{50}$) [µm] | 50 |
| Wassergehalt (Gew.-%, bezogen auf das Gesamtgewicht an Siral® 40) | n.b. |
| BET Oberfläche ($m^2/g$) | 512 |
| Kumulatives Porenvolumen (BJH) für Porendurchmesser 1.7 - 300 nm ($cm^3/g$) | 0,97 |
| Durchschnittlicher Porendurchmesser (BJH) (nm) | 7,15 |
| Zetapotential in Acetatpuffer pH 5,6 | - 15,2 mV |
| *) LOI: Glühverlust<br>n.b.: nicht bestimmt | |

Zu Vergleichszwecken wurde zudem die Urease-Inhibierung in Gegenwart von 150 mg Siral® 40 beziehungsweise in Gegenwart von 50 mg Zinkacetat ermittelt. Die Blindwertmessung mit reinem Siral® 40 zeigt keine Inhibierung von Urease. Durch Verwendung von belegtem Siral® 40 zeigt sich nur eine geringe Ammoniak-Bildung. Bei Verwendung von belegtem Siral® 40 erkennt man eine starke Inhibierung.

In einer Vergleichsmessung mit 50 mg reinem Zinkacetat entsteht eine vergleichsweise größere Menge an Ammoniak. Trotz eines hohen Zinküberschusses durch Verwendung von 50 mg reinem Zinkacetat zeigt sich bei der Verwendung des belegten Siral® 40 eine stärkere Inhibierung, was auf den Effekt der Urease-Adsorption, sowie einer Urease-Inhibierung durch Träger-gebundenes Zink hindeuten kann.

e) Zinkacetat belegtes Sephadex QAE

Weitere Versuche wurden unter Verwendung von mit Zinkacetat belegtem Sephadex® QAE (registered trademark of CGE Health Care, bezogen über Sigma Aldrich, Taufkirchen, Deutschland) durchgeführt. Dabei handelt es sich um Diethyl-(2-hydroxypropyl)aminoethyl Sephadex®, d.h. um einen Anionenaustauscher auf der Basis von Dextran. Es wurde eine gemäß Beispiel 1b durchgeführte Messung vorgenommen.

Bei einer Verwendung von mit Zinkacetat belegtem Sephadex QAE entsteht nur noch eine vernachlässigbar geringe Menge Ammoniak.

Beispiel 2:

Versuchsreihe 2: Urease-Inhibierung durch mit Zinkionen behandelte Träger in Gegenwart eines Superabsorbers

a) Vorbereitung der Träger

In einem 20 ml Becherglas werden 5 g Träger eingewogen. Anschließend werden 10 ml Wasser und 2 ml einer 20 Gew.%-igen Zinkacetat-Lösung zugegeben. Nach einer Standzeit von 16 h werden die Träger in einem Faltenfilter abfiltriert und jeweils mit 800 ml Wasser gewaschen. Dann werden die Träger bei 70˚C im Trockenschrank getrocknet.

b) Messung

1 g behandelter Träger, der gemäß Beispiel 2a hergestellt wurde, sowie 0,1 g Superabsorber (Polyacrylsäure zur Verwendung in Windeln), werden in 40 ml 2 Gew.%-igen Harnstoff-Lösung eingewogen. Anschließend werden sofort 2 ml einer 2 Gew.%-igen Urease-Lösung (Urease von Carl-Roth, etwa 1 U/mg, f.d. Biochemie, Art.-Nr.: 7573.1) zugegeben und der Spannungsverlauf bei einer Messung mit nachstehend angegebenen Messsystem notiert und hieraus die Konzentration an Ammoniak in der Lösung ermittelt. Das Messsystem war ein Mehrparameter Messsystem KM2000 von Sensortechnik Meinsberg. Daran angeschlossen war eine EGA 133 pH-Elektrode sowie eine Orion 95-12 Ammonia-Elektrode von Thermo. Die Ergebnisse zeigen deutlich eine Inhibierung der Urease bzw. eine verzögerte Ammoniak-Bildung bei einer Verwendung der behandelten Träger.

Zu Vergleichszwecken wurde zudem die Urease-Inhibierung in Gegenwart von 5 mg Zinkoxid beziehungsweise 13 mg Zinkacetat ermittelt. Hierzu wurden Zinkoxid oder Zinkacetat, sowie 0,1 g Superabsorber, in 40 ml 2 Gew.-% Harnstoff-Lösung eingewogen. Anschließend werden sofort 2 ml einer 2 Gew.%-igen Urease-Lösung zugegeben und der Spannungsverlauf notiert.

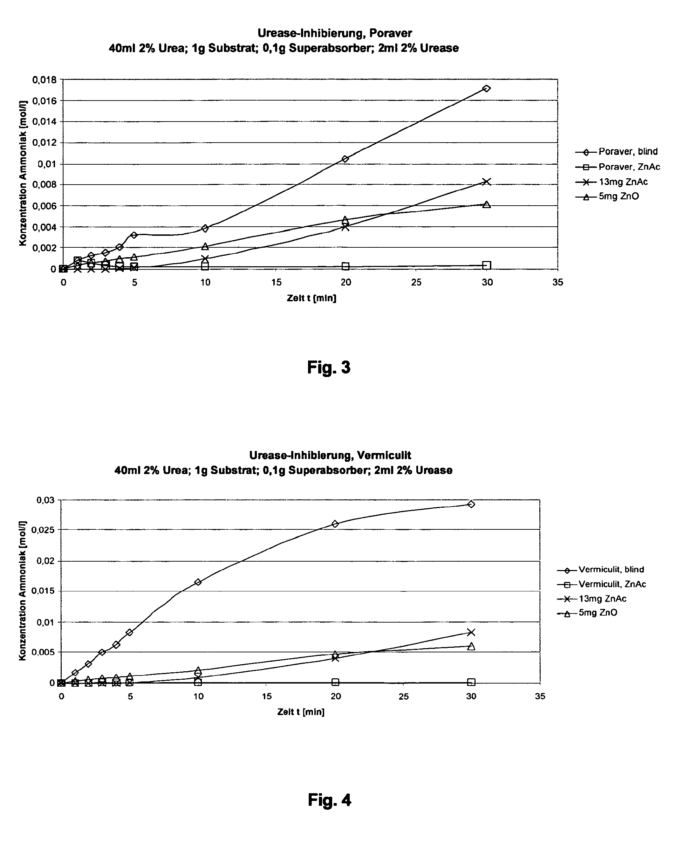

**Fig. 3** zeigt die Urease-Inhibierung in Gegenwart von 1 g mit Zinkacetat belegtem Poraver (Poraver® Glaskugeln (Poraver® Glaskugeln, Durchmesser, 0,1-0,3 mm, erhältlich über Dennert Poraver GmbH, Schlüsselfeld) bei einer gemäß Beispiel 2b durchgeführten Messung. Zu Vergleichszwecken wurde zudem die Urease-Inhibierung in Gegenwart von 1 g unbehandeltem Poraver ermittelt.

**Fig. 4** zeigt die Urease-Inhibierung in Gegenwart von 1 g mit Zinkacetat belegtem Vermiculit, Sorte Nr. 3 von Aldrich, D-82018 Taufkirchen bei einer gemäß Beispiel 2b durchgeführten Messung. Dieses Material ist relativ grob und wurde vor dem Einsatz nochmals weiter vermahlen. Zu Vergleichszwecken wurde zudem die Urease-Inhibierung in Gegenwart von 1 g unbehandeltem Vermiculit ermittelt. Vergleichsweise wurde der Vermiculit auch in der nicht vermahlenen Form eingesetzt. Dabei zeigte sich kein Unterschied in der Wirkung in der erfindungsgemäßen Formulierung. Für spätere technische Anwendungen, wie z.B. einen Einbau in Hygieneprodukte, kann jedoch die vermahlene Form des Vermiculits, beispielsweise aus herstellungstechnischen Gründen, bevorzugt sein.

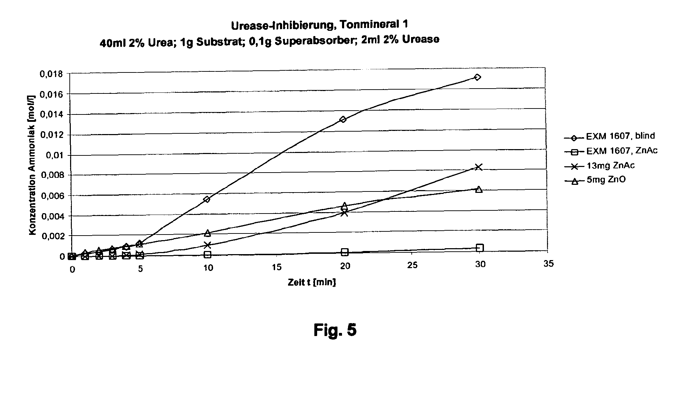

Vergleichbare Ergebnisse wie mit dem Vermiculit wurden auch mit einem natürlichen Tonmineral als Trägermaterial für die $Zn^{2+}$-Ionen gefunden (Tonmineral 1; **Fig. 5**). Das eingesetzte Material besteht aus einer natürlichen Mischung aus Kieselgel und smektitischem Ton (Bentonit). Durch den Einbau der Bentonitplättchen in die Kieselgelstruktur weist dieses Material nahezu kein Quellvermögen mehr auf, wie es für Bentonite typisch ist. Die charakteristischen Daten dieses Materials sind in den folgenden Tabellen aufgelistet.

Es wird angenommen, dass durch das Kationenaustauschvermögen dieses Materials Zinkionen gut auf die Oberfläche gebunden werden und für die Inhibierung der Urease zur Verfügung gestellt werden können.

**Tabelle 2:**

| Physikalische Eigenschaften von Tonmineral 1 (Kieselgel-Smektit-Mischphase) | |
|---|---|
| Physikalische Daten | |
| Trockensiebrückstand auf 45 $\mu$m (%) | 49 |
| Trockensiebrückstand auf 63 $\mu$m (%) | 35 |
| Schüttgewicht(g/1) | 292 |
| Methylenblauadsorption (mg/g Probe) | 106 |
| Wassergehalt (%) | 8 |
| pH (10 Gew.-% in Wasser) | 7.6 |
| Kationenaustauschkapazität (meq/100 g) | 52 |
| BET Oberfläche ($m^2$/g) | 208.4 |
| Kumulatives Porenvolumen (BJH) für Porendurchmesser 1.7 - 300 nm ($cm^3$/g) | 0.825 |
| Durchschnittlicher Porendurchmesser (BJH) (nm) | 16.4 |
| Sediment- bzw. Quellvolumen (ml/2g) | 5.5 |
| Zetapotential pH 7 | - 67 mV |

**Tabelle 3:**

| Silicatanalyse von Tonmineral 1 | |
|---|---|
| Oxid [Gew.-%] | |
| $SiO_2$ | 70.6 |
| $Fe_2O_3$ | 2.8 |
| $Al_2O_3$ | 9.8 |
| MgO | 4.1 |
| CaO | 1.4 |
| $K_2O$ | 1.5 |
| $Na_2O$ | 0.26 |
| $TiO_2$ | 0.25 |
| $SO_3$ | -- |
| GV *)(1000 ˚C) | 7.9 |
| *)GV: Glühverlust | |

Tabelle 4: mineralische Zusammensetzung von Tonmineral 1, ermittelt durch Auswertung von Röntgendiffraktogrammen unter Verwendung der Rietveld-Analyse

| Mineralische Phase | Gew % |
|---|---|
| Smektit (Gew.-%) | 40 |
| Illit / Muscovit (Gew.-%) | Spuren |
| Kaolinit (Gew.-%) | n.b. |

(fortgesetzt)

| Mineralische Phase | Gew % |
|---|---|
| Sepiolith (Gew.-%) | 11 |
| Quarz (Gew.-%) | Spuren |
| Orthoclas (Gew.-%) | 12 |
| Plagioclas (verschieden) (Gew.-%) | 3 |
| Calcit (Gew.-%) | Spuren |
| Amorphes Material (Gew.-%) | 34 |

**Patentansprüche**

1. Verwendung eines festen, teilchenförmigen Trägers zur Inhibierung der Aktivität von Urease in einem Urease enthaltenden fluiden Medium,
   wobei der feste teilchenförmige Träger mit mindestens einer Zinkionen und/oder Kupfer(II)-Ionen enthaltenden Lösung vor dem Inkontaktbringen mit dem Urease enthaltenden fluiden Medium behandelt wurde, und
   wobei die mindestens eine Zinkionen und/oder Kupfer(II)-Ionen enthaltende Lösung eine Lösung mindestens eines Zinksalzes und/oder mindestens eines Kupfer(II)-Salzes umfasst, wobei das mindestens eine Zinksalz und das mindestens eine Kupfer(II)-Salz frei von dreizähnigen oder mehr als dreizähnigen Chelat-Liganden sind.

2. Verwendung nach einem der vorstehenden Ansprüche, wobei das mindestens eine Zinksalz und/oder das mindestens eine Kupfer(II)-Salz jeweils eine Löslichkeit in Wasser von mehr als $10^{-10}$ mol/l aufweisen, wobei vorzugsweise die mindestens eine Zinkionen und/oder Kupfer(II)-Ionen enthaltende Lösung mindestens eine Zinkionen enthaltende Lösung ist, die eine Lösung mindestens einen Zinksalzes umfasst, das eine Löslichkeit in Wasser von mehr als $10^{-10}$ mol/l aufweist.

3. Verwendung nach einem der vorstehenden Ansprüche, **dadurch gekennzeichnet, dass** der teilchenförmige Träger Teilchen umfasst oder aus diesen besteht, die ein Zetapotential bei einem pH-Wert von 5 bis 9, insbesondere bei einem pH-Wert von 5,6, von weniger als 1 mV, vorzugsweise von weniger als -2,5 mV, bevorzugt von weniger als - 5 mV aufweisen.

4. Verwendung nach einem der vorstehenden Ansprüche, **dadurch gekennzeichnet, dass** der Träger eine Kationenaustauschkapazität von mehr als 10 meq pro 100g an Träger, vorzugsweise von mindestens 15 meq pro 100g an Träger, weiter bevorzugt von mindestens 25 meq pro 100g an Träger aufweist.

5. Verwendung nach einem der vorstehenden Ansprüche, **dadurch gekennzeichnet, dass** der Träger ein oder mehrere Materialien, ausgewählt aus der Gruppe bestehend aus $SiO_2$-haltigen Materialien, Titandioxiden, Aluminiumoxiden, Zinkoxiden, Zinksulfiden, Aktivkohlen, Holzkohlen, Zirkondioxiden, Polymeren, Cellulosen, Dextrane und deren Gemischen umfasst oder hieraus besteht.

6. Verwendung nach einem der vorstehenden Ansprüche, **dadurch gekennzeichnet, dass** das $SiO_2$-haltige Material ausgewählt ist, aus der Gruppe bestehend aus, porösen Glasmaterialien, Kieselsäuren, silikatischen Materialien, schichtsilikathaltiges Material, vorzugsweise mindestens ein phyllosilikathaltiges Material, Tonen, Bleicherden und Gemischen hiervon.

7. Verwendung nach einem der vorstehenden Ansprüche, **dadurch gekennzeichnet, dass** es sich bei dem silikatischen Material um ein Silica-Mischphasen umfassendes Material, insbesondere ein Bentonit-Silica-Mischphasen, umfassendes Material handelt.

8. Verwendung nach Anspruch 6, **dadurch gekennzeichnet, dass** das mindestens eine phyllosilikathaltige Material mindestens ein Phyllosilikat, ausgewählt aus der Gruppe bestehend aus Phyllosilikaten aus der Klasse der Smektite, Phyllosilikaten aus der Klasse der Vermiculite, Phyllosilikaten aus der Klasse der Glimmer und deren Gemischen umfasst oder hieraus besteht, wobei es sich vorzugsweise bei dem Phyllosilikat aus der Klasse der Smektite um einen Montmorillonit, insbesondere Bentonit, Vermiculit, Attapulgit, Saponit, Stevensit, Beidellit oder Gemische hiervon handelt.

9. Verwendung nach einem der vorstehenden Ansprüche, **dadurch gekennzeichnet, dass** die Behandlung des festen teilchenförmigen Trägers mit der mindestens einen Zinkionen und/oder Kupfer(II)-Ionen enthaltenden Lösung durch Aufsprühen der mindestens einen Zinkionen und/oder Kupfer(II)-Ionen enthaltenden Lösung, durch Tränken mit der mindestens einen Zinkionen und/oder Kupfer(II)-Ionen enthaltenden Lösung oder durch Suspendieren des festen teilchenförmigen Trägers in der mindestens einen Zinkionen und/oder Kupfer(II)-Ionen enthaltenden Lösung erfolgt.

10. Verwendung nach einem der vorstehenden Ansprüche, **dadurch gekennzeichnet, dass** der feste teilchenförmige Träger kein Carboxylgruppen aufweisendes Polymer darstellt oder dieses umfasst.

11. Verwendung nach einem der vorstehenden Ansprüche, **dadurch gekennzeichnet, dass** die Verwendung in einem Produkt erfolgt, das mindestens ein Absorbens enthält, wobei das mindestens eine Absorbens vorzugsweise mindestens einen Superabsorber umfasst, wobei der mindestens eine Superabsorber vorzugsweise ausgewählt ist, aus der Gruppe, bestehend aus Polyacrylsäuren, Polyacrylaten, Carboxy-Cellulosen, insbesondere Carboxymethylcellulosen, und deren Gemischen.

12. Verwendung nach einem der vorstehenden Ansprüche, wobei die Inhibierung der Aktivität von Urease in einem Urease enthaltenden Medium in Gegenwart eines Absorbens, insbesondere eines Superabsorbers erfolgt.

13. Verwendung nach einem der vorstehenden Ansprüche zur Verhinderung oder Verringerung einer Geruchsbelastung, insbesondere eines Geruchs von Ammoniak, aus urinhaltigen fluiden Medien, insbesondere Urin oder urinhaltigen Gemischen.

14. Verwendung nach einem der vorstehenden Ansprüche zur Inhibierung der Aktivität von Urease in einem absorbierenden Artikel, insbesondere Windel, Windeleinlage, Binde, Einlage, Tuch, Betteinlage, Inkontinenzartikel, sowie Haustierstreu, und/oder
zur Verhinderung oder Verringerung einer Geruchsentwicklung, insbesondere einer Ammoniak-Geruchsentwicklung, in einem absorbierenden Artikel nach in Kontakt kommen mit einem urinhaltigen fluiden Medium, und/oder
zur Inhibierung der Aktivität von Urease in Viehställen, Katzenklos oder Tierkäfigen und/oder
zur Verhinderung oder Verringerung eines Abbaus von Harnstoff basiertem Dünger durch Reaktion mit Urease.

15. Verfahren zur Herstellung einer Ureaseinhibitor-Träger-Zusammensetzung, umfassend die Schritte:

   a. Bereitstellen mindestens eines festen teilchenförmigen Trägers,
   b. Bereitstellen mindestens einer Zinkionen und/oder Kupfer(II)-Ionen enthaltenden Lösung,
   c. Behandeln des mindestens einen festen teilchenförmigen Trägers mit der mindestens einen Zinkionen und/oder Kupfer(II)-Ionen enthaltenden Lösung, vorzugsweise in Abwesenheit eines Urease enthaltenden fluiden Mediums; und
   d. optional Trocknen des gemäß Schritt c. behandelten festen teilchenförmigen Trägers.

16. Absorbierender Artikel, insbesondere Windel, Hygienebinde, Windeleinlage, Binde, Einlage, Tuch, Betteinlage, Inkontinenzartikel, Haustierstreu, Streu zum Ausbringen in Nutztierställen, umfassend,
mindestens ein Absorbens, insbesondere mindestens einen Superabsorber, wobei der Superabsorber vorzugsweise ausgewählt ist, aus der Gruppe, bestehend aus Polyacrylaten, Polyacrylsäuren, Carboxy-Cellulosen, insbesondere Carboxymethylcellulosen, und deren Gemischen, sowie einen mit mindestens einer Zinkionen und/oder Kupfer (II)-Ionen enthaltenden Lösung behandelten festen teilchenförmigen Träger.

**Urease-Inhibierung, Vergleich ZnO-Adsorbentien**

Fig.1

**Urease-Inhibierung, Vergleich Siral® 40**

Fig. 2

**Urease-Inhibierung, Poraver**
**40ml 2% Urea; 1g Substrat; 0,1g Superabsorber; 2ml 2% Urease**

Konzentration Ammoniak [mol/l]

Zeit t [min]

Poraver, blind
Poraver, ZnAc
13mg ZnAc
5mg ZnO

**Fig. 3**

**Urease-Inhibierung, Vermiculit**
**40ml 2% Urea; 1g Substrat; 0,1g Superabsorber; 2ml 2% Urease**

Konzentration Ammoniak [mol/l]

Zeit t [min]

Vermiculit, blind
Vermiculit, ZnAc
13mg ZnAc
5mg ZnO

**Fig. 4**

Urease-Inhibierung, Tonmineral 1
40ml 2% Urea; 1g Substrat; 0,1g Superabsorber; 2ml 2% Urease

**Fig. 5**

**EP 2 198 892 A1**

Europäisches Patentamt
European Patent Office
Office européen des brevets

# EUROPÄISCHER RECHERCHENBERICHT

Nummer der Anmeldung

EP 08 02 1914

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2005/084438 A1 (DO BAO T [US] ET AL DO BAO TRONG [US] ET AL) 21. April 2005 (2005-04-21) * Seite 1, Absatz 3 - Absatz 7 * * Seite 2, Absatz 17 * * Seite 3, Absatz 19 * * Beispiele 1-8 * * Ansprüche 1-11 * | 1-6,9-16 | INV. A61L15/18 A61L15/46 A61L15/60 |
| X | DE 199 29 106 A1 (FORMOSA JOHN PATRICK [AU]; PFEIFER URSULA BRIGITTE [DE]) 28. Dezember 2000 (2000-12-28) * Spalte 1, Zeile 26 - Zeile 65 * * Ansprüche 1,2,4 * | 1-16 | |
| X | EP 1 632 253 A1 (BKI HOLDING CORP [US]) 8. März 2006 (2006-03-08) * Seite 2, Absatz 8 - Seite 3, Absatz 14 * * Seite 13, Absatz 65 - Seite 14, Absatz 66 * * Seite 14, Absatz 71 - Seite 15, Absatz 72 * * Seite 15, Absatz 77 * * Ansprüche 1-14,31 * | 1-3,5, 9-16 | RECHERCHIERTE SACHGEBIETE (IPC) A61L |
| X | WO 2006/107980 A2 (ECO PETS LLC [US]; COOPER ROBERT [US]; WHEELER PETER [US]; DIPAOLO DAN) 12. Oktober 2006 (2006-10-12) * Seite 3, Zeile 26 - Seite 4, Zeile 9 * * Seite 8, Zeile 30 - Seite 10, Zeile 15 * * Ansprüche 19,32,33 * | 1-4, 10-16 | |

-/--

Der vorliegende Recherchenbericht wurde für alle Patentansprüche erstellt

| Recherchenort | Abschlußdatum der Recherche | Prüfer |
|---|---|---|
| Den Haag | 3. Juni 2009 | Menidjel, Razik |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes Dokument

EPO FORM 1503 03.82 (P04C03)

24

**Europäisches Patentamt**
**European Patent Office**
**Office européen des brevets**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 95/24173 A2 (PROCTER & GAMBLE [US]) 14. September 1995 (1995-09-14) * Seite 1, Zeile 7 - Zeile 12 * * Seite 2, Zeile 20 - Seite 3, Zeile 17 * * Seite 4, Zeile 21 - Seite 5, Zeile 19 * * Seite 9, Zeile 1 - Zeile 20 * * Ansprüche 1,2,3,4,5,6,8 * ----- | 1-6,9-16 | |
| X | FR 2 793 386 A1 (CECA SA [FR]) 17. November 2000 (2000-11-17)  * Seite 1, Zeile 5 - Zeile 9 * * Seite 1, Zeile 34 - Seite 2, Zeile 23 * * Seite 3, Zeile 19 - Zeile 24 * * Ansprüche 1-11 * ----- | 1-6, 9-10, 13-16 | |
| X | EP 0 389 023 A2 (PROCTER & GAMBLE [US]) 26. September 1990 (1990-09-26) * Seite 3, Zeile 46 - Seite 4, Zeile 2 * * Seite 7, Zeile 45 - Seite 8, Zeile 4 * * Seite 9, Zeile 39 - Seite 10, Zeile 17 * * Seite 11, Zeile 19 - Zeile 27 * * Beispiel 4 * * Ansprüche 1-4 * ----- | 1-2,5, 9-16 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Den Haag | 3. Juni 2009 | Menidjel, Razik |

**ANHANG ZUM EUROPÄISCHEN RECHERCHENBERICHT**
**ÜBER DIE EUROPÄISCHE PATENTANMELDUNG NR.**

EP 08 02 1914

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-06-2009

| Im Recherchenbericht angeführtes Patentdokument | | Datum der Veröffentlichung | Mitglied(er) der Patentfamilie | | | Datum der Veröffentlichung |
|---|---|---|---|---|---|---|
| US 2005084438 | A1 | 21-04-2005 | US | 2009054859 | A1 | 26-02-2009 |
|  |  |  | WO | 2005039655 | A1 | 06-05-2005 |
| DE 19929106 | A1 | 28-12-2000 | KEINE | | | |
| EP 1632253 | A1 | 08-03-2006 | CA | 2576045 | A1 | 16-02-2006 |
|  |  |  | WO | 2006017441 | A1 | 16-02-2006 |
| WO 2006107980 | A2 | 12-10-2006 | KEINE | | | |
| WO 9524173 | A2 | 14-09-1995 | AT | 194278 | T | 15-07-2000 |
|  |  |  | AU | 1932195 | A | 25-09-1995 |
|  |  |  | CA | 2185107 | C | 05-12-2000 |
|  |  |  | DE | 69517799 | D1 | 10-08-2000 |
|  |  |  | DE | 69517799 | T2 | 01-02-2001 |
|  |  |  | EP | 0749295 | A1 | 27-12-1996 |
|  |  |  | ES | 2149973 | T3 | 16-11-2000 |
|  |  |  | HK | 1012939 | A1 | 30-03-2001 |
|  |  |  | JP | 9509870 | T | 07-10-1997 |
|  |  |  | US | H1732 | H | 02-06-1998 |
| FR 2793386 | A1 | 17-11-2000 | KEINE | | | |
| EP 0389023 | A2 | 26-09-1990 | AU | 620225 | B2 | 13-02-1992 |
|  |  |  | AU | 5146490 | A | 20-09-1990 |
|  |  |  | BR | 9001300 | A | 02-04-1991 |
|  |  |  | CA | 2011670 | A1 | 20-09-1990 |
|  |  |  | CN | 1046093 | A | 17-10-1990 |
|  |  |  | JP | 3000058 | A | 07-01-1991 |
|  |  |  | MA | 21775 | A1 | 01-10-1990 |
|  |  |  | NZ | 232976 | A | 26-05-1993 |
|  |  |  | PT | 93466 | A | 07-11-1990 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20030220211 A1
- WO 0247472 A
- DE 10016488 A1
- WO 2007085531 A2
- EP 0391108 A2

### In der Beschreibung aufgeführte Nicht-Patentliteratur

- **Holleman-Wiberg.** Lehrbuch der Anorganischen Chemie. Walter de Gruyter & Co, 1985, 969-983
- **E. Riedel.** Anorganische Chemie. Walter de Gruyter& Co, 1988, 597-606
- **Juan M. Garcia-Segura.** Sepiolite-supported Urease. *Concepción Cid*
- **Javier Martin de Llano ; José G. Gavilanes.** *British Polymer Journal,* 1987, vol. 19, 517-522
- **Günay Demirel ; Güneri Akovali ; Abdurrahman Tanyolac ; Nesrin Hasirci.** A Comparative Study of the Performance of Solid Supported and Soluble Urease for the Enzymatic Hydrolysis of Urea. *J. Chem. Tech. Biotechnol.,* 1992, vol. 55, 319-323
- Immobilization of urease on vermiculite. **A. Anita ; C.A. Sastry ; M.A. Hashim.** Bioprocess Engineering. Springer-Verlag, 1997, vol. 16, 375-380
- Immobilization of urease using Amberlite MB-1. **A. Anita ; C.A. Sastry ; M.A. Hashim.** Bioprocess Engineering. Springer-Verlag, 1997, vol. 17, 355-359
- Dispersionen und Emulsionen, eine Einführung in die Kolloidik fein verteilter Teilchen einschließlich der Tonminerale. **G. Lagaly ; O. Schulz ; R. Zimehl.** Oberflächenpotential und elektrokinetisches Potential. Dr. Dietrich Steinkopff-Verlag, 1997, 347 ff
- **R.A. Young.** The Rietveld Method. Oxford University Press, 1995
- **D.K. McCarthy.** Quantitative Mineral Analysis of Clay-bearing Mixtures. *The Reynolds Cup,* 2002, vol. 27, 12-16
- Hand Book of Clay Science. Elsevier, 2006
- X-Ray Diffraction and the Identification and Analysis of Clay Minerals. **I. Srodon.** Identification and Quantitative Analysis of Clay Minerals. Oxford University Press, 1997, 765
- **Holleman-Wiberg.** Lehrbuch der Anorganischen Chemie. Walter de Gruyter & Co, 1985, 768-779
- *J. Am. Chem. Soc.,* 1938, vol. 60, 309
- **E.P. Barrett ; L.G. Joyner ; P.P. Hienda.** *J. Am. Chem. Soc.,* 1951, vol. 73, 373
- **Brindley ; Brown.** Crystal structures of clay minerals and their x-ray identification. *Mineralogical Society,* 1980, 495